# EUROPEAN PATENT APPLICATION

(11) **EP 4 464 301 A1**
(43) Date of publication of application: **20.11.2024**
(21) Application number: 23740345.6
(22) Date of filing: 13.01.2023
(51) Int. Cl.: A61K 6/30, A61K 6/62, A61K 6/71, A61K 6/896

(54) **DENTAL COMPOSITION, METHOD FOR MANUFACTURING SAME, AND DENTAL SURFACE-TREATED FILLER**

(30) Priority: 14.01.2022 JP 2022004712
(71) Applicant: Kuraray Noritake Dental Inc., Kurashiki-shi, Okayama 710-0801 (JP)
(72) Inventor: MATSUURA Ryo, Tainai-shi, Niigata 959-2653 (JP); SAKAMAKI Hiroshi, Kitakatsushika-gun, Saitama 345-0043 (JP); OBATA Yuki, Kitakatsushika-gun, Saitama 345-0043 (JP)
(74) Representative: D Young & Co LLP
(86) International application number: PCT/JP2023/000891
(87) International publication number: WO 2023/136337

(57) **Abstract**

The present invention provides a dental composition that exhibits excellent storage stability even under severe conditions, and shows sufficient mechanical strength in its cured product. The invention also provides a method of production of such dental compositions, and surface-treated fillers for dental use. The present invention relates to a dental composition comprising a polymerizable monomer (A), a polymerization initiator (B), and a filler (C), wherein the filler (C) comprises a surface-treated filler for dental use (C-1) surface-treated with a silane coupling agent (a) having a polymerizable group, and an end capping agent (b), and the surface-treated filler for dental use (C-1) shows a dissolution rate of less than 100 ppm for metallic components in an acid resistance test.

## Description

### TECHNICAL FIELD

The present invention relates to dental compositions. More specifically, the invention relates to a dental composition that exhibits excellent storage stability even under severe conditions, and shows sufficient mechanical strength in its cured product. The invention also relates to a method of production of such dental compositions, and surface-treated fillers for dental use.

### BACKGROUND ART

In the treatment of tooth decay and associated defects, traditional restorative treatment typically involves using dental bonding materials and dental composite resins. The procedure for such restorative treatment follows these steps. First, the damaged or decayed area is removed to create a cavity, and a dental bonding material is applied to the cavity. After optionally removing the solvent using techniques such as air blowing, the dental bonding material is cured by applying visible light over the areas including the dental bonding material. Subsequently, a dental composite resin is filled onto the cured layer of dental bonding material, and is cured by exposure to visible light.

This restorative treatment uses two materials: dental bonding material and dental composite resin. Over the last years, however, a self-adhesive dental composite resin has been developed that incorporates adhesive properties in the dental composite resin itself. This material, by eliminating the need for dental bonding material, is finding practical applications in restorative treatment involving fewer steps.

Self-adhesive dental composite resins contain polymerizable monomers having acidic groups such as phosphoric acid groups or carboxy groups-a material used for dental bonding materials or other applications-to provide and enhance self-adhesive properties to surfaces such as tooth structure, in addition to containing components found in conventional dental composite resins, such as polyfunctional polymerizable monomers, fillers, and polymerization initiators (see, for example, Patent Literature 1).

When materials such as oxides, carbonates, or hydroxides of alkali earth metals such as barium and strontium, or acid-reactive fluoroaluminosilicate glass, typically used as fillers in dental composite resins, are incorporated into self-adhesive dental composite resins such as that disclosed in Patent Literature 1, it induces acid-base reaction, neutralization, salt formation, or chelation reaction between the fillers and polymerizable monomers having acidic groups. Because these reactions consume the polymerizable monomers having acidic groups, there exists an issue between these specific fillers and the polymerizable monomers having acidic groups, specifically, loss of self-adhesive properties.

This issue is commonly observed in dental curable compositions that contain these specific fillers and polymerizable monomers having acidic groups. Various dental curable compositions have been proposed to address this issue (see, for example, Patent Literatures 2 to 4). Patent Literature 2 discloses a single-component self-adhesive dental composition comprising a filler with low reactivity toward acidic components, for example, such as a silica filler treated with a silane coupling agent.

Patent Literature 3 discloses a dental composition with superior storage stability, achieved by reducing the amount of polyvalent metals on inorganic particle surfaces through an acid treatment process of inorganic particles containing polyvalent metals, despite containing polymerizable monomers having acidic groups, and inorganic particles containing polyvalent metals. Additionally, Patent Literature 4 discloses incorporating inorganic particles surface-treated with silane coupling agents and organosilazanes, both with specific structures, providing a self-adhesive dental composite resin that exhibits superior storage stability while maintaining mechanical strength, and that undergoes reduced alterations in paste transparency and properties, and carries a low risk of solidification during prolonged storage.

### CITATION LIST

### Patent Literature

Patent Literature 1: JP 2008-260752 A
Patent Literature 2: JP 2015-507610 T
Patent Literature 3: JP 2011-178778 A
Patent Literature 4: WO2018/074594

### SUMMARY OF INVENTION

### Technical Problem

However, upon examination by the present inventors, it was found that sufficient storage stability is unachievable with the single-component self-adhesive dental composition of Patent Literature 2 comprising, for example, a silica filler that has been subjected to surface treatment with a common silane coupling agent.

The acid treatment disclosed in Patent Literature 3 exhibits some effect on storage stability. However, further improvements are needed under more severe conditions (for example, 60°C for 4 weeks).

Concerning the specific surface treatment disclosed in Patent Literature 4, the surface treatment of silica fillers produces a relatively superior effect in terms of paste properties such as paste transparency in relation to the storage stability of the self-adhesive dental composite resin containing the surface-treated silica filler. However, this related art is silent as to the adhesive properties after prolonged storage. The present inventors have found that further improvements are needed in storage stability following investigation involving application of similar treatment to X-ray opacity fillers used to provide the X-ray opacity characteristics necessary in materials such as dental composite resins and dental cements.

It is accordingly an object of the present invention to provide a dental composition that exhibits excellent storage stability even under severe conditions, and shows sufficient mechanical strength in its cured product. Another object of the invention is to provide a method of production of such dental compositions, and surface-treated fillers for dental use.

### Solution to Problem

Specifically, the present invention includes the following.
[1] A dental composition comprising a polymerizable monomer (A), a polymerization initiator (B), and a filler (C),
   wherein the filler (C) comprises a surface-treated filler for dental use (C-1) surface-treated with a silane coupling agent (a) having a polymerizable group, and an end capping agent (b), and
   the surface-treated filler for dental use (C-1) shows a dissolution rate of less than 100 ppm for metallic components in an acid resistance test.
[2] The dental composition according to [1], wherein the end capping agent (b) is a compound having a structure represented by the following formula (1) or (2), and a boiling point of 180°C or less, 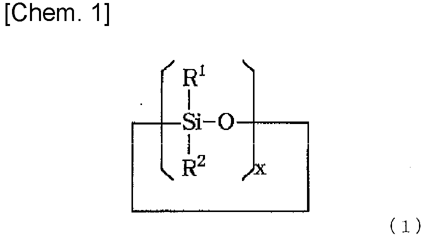 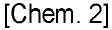 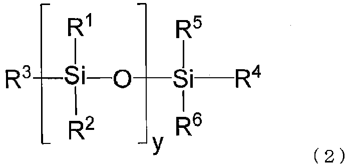 where R¹ to R⁶ each independently represent a hydrogen atom, a hydroxyl group, an alkyl group having 1 to 9 carbon atoms, or an alkoxy group having 1 to 4 carbon atoms, x represents an integer of 3 to 10, and y represents an integer of 1 to 100.
[3] The dental composition according to [1] or [2], wherein the surface-treated filler for dental use (C-1) results from surface treatment with the end capping agent (b) following surface treatment with the silane coupling agent (a) having a polymerizable group.
[4] The dental composition according to any one of [1] to [3], wherein the surface-treated filler for dental use (C-1) results from hydrolysis following surface treatment with the silane coupling agent (a) having a polymerizable group.
[5] The dental composition according to any one of [1] to [4], wherein the surface-treated filler for dental use (C-1) comprises an X-ray opacity filler.
[6] The dental composition according to [5], wherein the X-ray opacity filler results from acid treatment.
[7] The dental composition according to any one of [1] to [6], wherein the silane coupling agent (a) having a polymerizable group comprises 3-methacryloyloxypropyltrimethoxysilane, 8-(meth)acryloyloxyoctyltrimethoxysilane, 11-(meth)acryloyloxyundecyltrimethoxysilane, and hydrolysates of these.
[8] The dental composition according to any one of [1] to [7], wherein the end capping agent (b) is hexamethylcyclotrisiloxane.
[9] The dental composition according to any one of [1] to [8], wherein a filler is treated with 0.01 to 20 parts by mass of the end capping agent (b) in surface treatment to obtain the surface-treated filler for dental use (C-1), relative to 100 parts by mass of the filler before surface treatment.
[10] The dental composition according to any one of [1] to [9], wherein the polymerizable monomer (A) comprises a polymerizable monomer (A-1) having an acidic group, and a polymerizable monomer (A-2) having no acidic group.
[11] The dental composition according to [10], wherein the content of the polymerizable monomer (A-1) having an acidic group is 1 to 40 parts by mass in 100 parts by mass of the polymerizable monomer (A).
[12] A self-adhesive dental composite resin comprising a dental composition of any one of [1] to [11].
[13] A dental bonding material comprising a dental composition of any one of [1] to [11].
[14] A dental cement comprising a dental composition of any one of [1] to [11].
[15] A dental composite resin comprising a dental composition of any one of [1] to [11].
[16] A dental abutment construction material comprising a dental composition of any one of [1] to [11].
[17] A method for producing a dental composition of any one of [1] to [11], comprising a step of obtaining the surface-treated filler for dental use (C-1), wherein the step of obtaining the surface-treated filler for dental use (C-1) comprises subjecting a filler to surface treatment with the silane coupling agent (a) having a polymerizable group, and subjecting the filler to surface treatment with the end capping agent (b).
[18] A surface-treated filler for dental use (C-1) that results from surface treatment of filler with a silane coupling agent (a) having a polymerizable group, and an end capping agent (b),
   wherein the end capping agent (b) is a compound having a structure represented by the following formula (1) or (2), and a boiling point of 180°C or less, 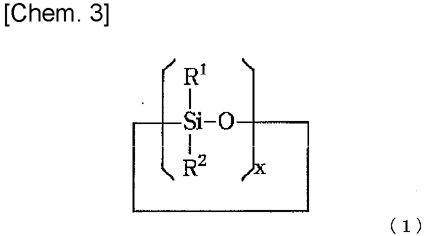 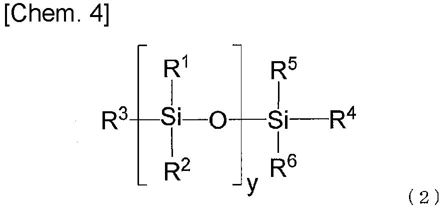 where R¹ to R⁶ each independently represent a hydrogen atom, a hydroxyl group, an alkyl group having 1 to 9 carbon atoms, or an alkoxy group having 1 to 4 carbon atoms, x represents an integer of 3 to 10, and y represents an integer of 1 to 100.
[19] The surface-treated filler for dental use (C-1) according to [18], wherein the filler comprises an inorganic filler.
[20] The surface-treated filler for dental use (C-1) according to [19], wherein the inorganic filler comprises an aggregate filler.
[21] The surface-treated filler for dental use (C-1) according to any one of [18] to [20], which results from surface treatment with the end capping agent (b) following surface treatment with the silane coupling agent (a) having a polymerizable group.
[22] The surface-treated filler for dental use (C-1) according to any one of [18] to [21], which results from hydrolysis following surface treatment with the silane coupling agent (a) having a polymerizable group.

### Advantageous Effects of Invention

According to the present invention, a dental composition can be provided that exhibits excellent storage stability even under severe conditions, and shows sufficient mechanical strength in its cured product. The invention can also provide a method of production of such dental compositions, and surface-treated fillers for dental use.

With such outstanding properties, a dental composition of the present invention can be suitably used in applications such as self-adhesive dental composite resins, dental bonding materials, dental cements, dental composite resins, and dental abutment construction materials.

### BRIEF DESCRIPTION OF DRAWINGS

[FIG. 1] A diagram representing the surface of the surface-treated filler used for the dental composition according to Comparative Example 1-1.
[FIG. 2] A diagram representing the surface of the surface-treated filler used for the dental composition according to Example 1-1.

### DESCRIPTION OF EMBODIMENTS

A dental composition of the present invention comprises a polymerizable monomer (A), a polymerization initiator (B), and a filler (C),
wherein the filler (C) comprises a surface-treated filler for dental use (C-1) surface-treated with a silane coupling agent (a) having a polymerizable group, and an end capping agent (b), and
the surface-treated filler for dental use (C-1) shows a dissolution rate of less than 100 ppm for metallic components in an acid resistance test.

The end capping agent (b) is preferably a compound having a specific structure (described below), and a boiling point of 180°C or less.

In this specification, "(meth)acryl" is a collective term for methacryl and acryl. The same applies to similar expressions (such as "(meth)acrylic acid" and "(meth)acrylonitrile"). In this specification, the upper limits and lower limits of numeric ranges (for example, ranges of contents of components, ranges of values calculated from components, and ranges of physical properties) can be appropriately combined.

While the reasons why a dental composition of the present invention exhibits excellent storage stability even under severe conditions (for example, 60°C for 4 weeks), and shows sufficient mechanical strength in its cured product remain unclear, the following speculation has been made.

First, the reaction rate increases when the end capping agent (b) having a specific structure and a boiling point of 180°C or less is used to react with the filler in the gas phase, compared to reactions with other surface treatment agents and reactions occurring in the liquid phase. Additionally, using the end capping agent (b) with a boiling point of 180°C or less enables the reaction with the filler surface to occur at temperatures that do not deactivate the polymerizable groups. Consequently, the surface-treated filler (C-1), treated with the silane coupling agent (a) having a polymerizable group and the end capping agent (b) having a specific structure and a boiling point of 180°C or less, appears to have a denser coverage with organic molecules on its surface compared to surface-treated fillers treated with traditional techniques, while sufficiently maintaining polymerizable groups on the surface to ensure mechanical strength. It is believed that this prevents interactions between the surface-treated filler (C-1) and other constituent components, maintaining outstanding storage stability over extended time periods.

The following describes the components used for a dental composition of the present invention.

### <Polymerizable Monomer (A)>

Radical polymerizable monomers are suitably employed as the polymerizable monomer (A) used in a dental composition of the present invention. Specific examples of radical polymerizable monomers as polymerizable monomers (A) include (meth)acrylate polymerizable monomers, (meth)acrylamide polymerizable monomers, esters, vinyl esters, and vinyl ethers of acids such as α-cyanoacrylic acid, (meth)acrylic acid, α-halogenated acrylic acid, crotonic acid, cinnamic acid, sorbic acid, maleic acid, and itaconic acid, mono-N-vinyl derivatives, and styrene derivatives. Preferred in view of curability are (meth)acrylate polymerizable monomers, and (meth)acrylamide polymerizable monomers. In view of adhesive properties to tooth structure and elastic modulus, it is preferable in a dental composition of the present invention that the polymerizable monomer (A) comprise a polymerizable monomer (A-1) having an acidic group, and a polymerizable monomer (A-2) having no acidic group.

### . Polymerizable monomer (A-1) having an acidic group

The polymerizable monomer (A-1) having an acidic group is preferable for imparting adhesive properties to tooth structure. Examples of the polymerizable monomer (A-1) having an acidic group used in the present invention include polymerizable monomer having at least one acidic group such as a phosphoric acid group, a pyrophosphoric acid group, a thiophosphoric acid group, a phosphonic acid group, a carboxylic acid group, and a sulfonic acid group. The polymerizable monomer (A-1) having an acidic group may be used alone, or two or more thereof may be used in appropriate combinations. Specific examples of the polymerizable monomer (A-1) having an acidic group are as follows.

Examples of polymerizable monomers having a phosphoric acid group include 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, bis[2-(meth)acryloyloxyethyl]hydrogen phosphate, bis[4-(meth)acryloyloxybutyl]hydrogen phosphate, bis[6-(meth)acryloyloxyhexyl]hydrogen phosphate, bis[8-(meth)acryloyloxyoctyl]hydrogen phosphate, bis[9-(meth)acryloyloxynonyl]hydrogen phosphate, bis[10-(meth)acryloyloxydecyl]hydrogen phosphate, 1,3-di(meth)acryloyloxypropyl-2-dihydrogen phosphate, 2-(meth)acryloyloxyethylphenyl hydrogen phosphate, 2-(meth)acryloyloxyethyl-(2-bromoethyl)hydrogen phosphate, 2-methacryloyloxyethyl-(4-methoxyphenyl)hydrogen phosphate, 2-methacryloyloxypropyl-(4-methoxyphenyl)hydrogen phosphate, and acid chlorides, alkali metal salts, and amine salts of these.

Examples of polymerizable monomers having a pyrophosphoric acid group include bis[2-(meth)acryloyloxyethyl]pyrophosphate, bis[4-(meth)acryloyloxybutyl]pyrophosphate, bis[6-(meth)acryloyloxyhexyl]pyrophosphate, bis[8-(meth)acryloyloxyoctyl]pyrophosphate, bis[10-(meth)acryloyloxydecyl]pyrophosphate, and acid chlorides, alkali metal salts, and amine salts of these.

Examples of polymerizable monomers having a thiophosphoric acid group include 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphate, 6-(meth)acryloyloxyhexyl dihydrogen thiophosphate, 7-(meth)acryloyloxyheptyl dihydrogen thiophosphate, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate, 11-(meth)acryloyloxyundecyl dihydrogen thiophosphate, 12-(meth)acryloyloxydodecyl dihydrogen thiophosphate, 16-(meth)acryloyloxyhexadecyl dihydrogen thiophosphate, 20-(meth)acryloyloxyicosyl dihydrogen thiophosphate, and acid chlorides, alkali metal salts, and ammonium salts of these.

Examples of polymerizable monomers having a phosphonic acid group include 2-(meth)acryloyloxyethylphenyl phosphonate, 5-(meth)acryloyloxypentyl-3-phosphonopropionate, 6-(meth)acryloyloxyhexyl-3-phosphonopropionate, 1 0-(meth)acryloyloxydecyl-3-phosphonopropionate, 6-(meth)acryloyloxyhexyl-3-phosphonoacetate, 10-(meth)acryloyloxydecyl-3-phosphonoacetate, and acid chlorides, alkali metal salts, and ammonium salts of these.

Examples of polymerizable monomers having a carboxylic acid group include monofunctional (meth)acrylic acid esters having one carboxyl group or an acid anhydride group thereof within the molecule, and monofunctional (meth)acrylic acid esters having multiple carboxyl groups or acid anhydride groups thereof within the molecule.

Examples of the monofunctional polymerizable monomers having one carboxyl group or an acid anhydride group thereof within the molecule include (meth)acrylic acid, N-(meth)acryloyl glycine, N-(meth)acryloyl aspartic acid, 2-(meth)acryloyloxyethyl hydrogen succinate, 2-(meth)acryloyloxyethyl hydrogen phthalate, 2-(meth)acryloyloxyethyl hydrogen malate, O-(meth)acryloyl tyrosine, N-(meth)acryloyl tyrosine, N-(meth)acryloyl phenylalanine, N-(meth)acryloyl-p-aminobenzoic acid, N-(meth)acryloyl-o-aminobenzoic acid, 2-(meth)acryloyloxybenzoic acid, 3-(meth)acryloyloxybenzoic acid, 4-(meth)acryloyloxybenzoic acid, N-(meth)acryloyl-5-aminosalicylic acid, N-(meth)acryloyl-4-aminosalicylic acid, and compounds derived by converting the carboxyl group of the aforementioned compounds into an acid anhydride group.

Examples of the monofunctional polymerizable monomers having multiple carboxyl groups or acid anhydride groups thereof within the molecule include 6-(meth)acryloyloxyhexane-1,1 -dicarboxylic acid, 9-(meth)acryloyloxynonane-1,1 -dicarboxylic acid, 10-(meth)acryloyloxydecane-1,1-dicarboxylic acid, 11-(meth)acryloyloxyundecane-1,1 -dicarboxylic acid, 12-(meth)acryloyloxydodecane-1,1-dicarboxylic acid, 13-(meth)acryloyloxytridecane-1,1-dicarboxylic acid, 4-(meth)acryloyloxyethyl trimellitate, 4-(meth)acryloyloxyethyl trimellitate anhydride, 4-(meth)acryloyloxybutyl trimellitate, 4-(meth)acryloyloxyhexyl trimellitate, 4-(meth)acryloyloxydecyl trimellitate, 2-(meth)acryloyloxyethyl-3'-(meth)acryloyloxy-2'-(3,4-dicarboxybenzoyloxy)propyl succinate, 6-(meth)acryloyloxyethylnaphthalene-1,2,6-tricarboxylic anhydride, 6-(meth)acryloyloxyethylnaphthalene-2,3,6-tricarboxylic anhydride, 4-(meth)acryloyloxyethylcarbonylpropionoyl-1,8-naphthalic anhydride, and 4-(meth)acryloyloxyethylnaphthalene-1,8-tricarboxylic anhydride.

Examples of polymerizable monomers having a sulfonic acid group include 2-sulfoethyl (meth)acrylate.

In view of providing favorable bond strength when used for the dental composition, it is preferable among these polymerizable monomers (A-1) having an acidic group to comprise polymerizable monomers having a phosphoric acid group, or polymerizable monomers having a carboxylic acid group, more preferably 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, 4-(meth)acryloyloxyethyl trimellitate anhydride, 4-(meth)acryloyloxyethyl trimellitate, 11-(meth)acryloyloxyundecane-1,1-dicarboxylic acid, and a mixture of 2-methacryloyloxyethyl dihydrogen phosphate and bis(2-methacryloyloxyethyl)hydrogen phosphate, even more preferably 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, and 20-(meth)acryloyloxyicosyl dihydrogen phosphate. In view of a balance with curability, 10-(meth)acryloyloxydecyl dihydrogen phosphate is most preferred.

In view of adhesive properties to tooth structure, the content of the polymerizable monomer (A-1) having an acidic group in a dental composition of the present invention is preferably 1 to 40 parts by mass, more preferably 2.5 to 35 parts by mass, even more preferably 5 to 30 parts by mass in 100 parts by mass of polymerizable monomer (A).

### · Polymerizable monomer (A-2) having no acidic group

Examples of the polymerizable monomer (A-2) having no acidic group in the present invention include a hydrophobic polymerizable monomer (A-2a) having no acidic group with a solubility of less than 10 mass% in 25°C water; and a hydrophilic polymerizable monomer (A-2b) having no acidic group with a solubility of 10 mass% or more in 25°C water. The polymerizable monomer (A-2) having no acidic group may be used alone, or two or more thereof may be used in combination.

### · Hydrophobic polymerizable monomer (A-2a) having no acidic group

The hydrophobic polymerizable monomer (A-2a) having no acidic group (hereinafter, also referred to simply as "hydrophobic polymerizable monomer (A-2a)") improves properties such as ease of handling of the dental composition, and the mechanical strength of the cured product.

Preferred as hydrophobic polymerizable monomer (A-2a) are radical polymerizable monomers having no acidic group but having a polymerizable group. In view of ease of radical polymerization, the polymerizable group is preferably a (meth)acryloyloxy group and/or a (meth)acrylamide group. Here, hydrophobic polymerizable monomer (A-2a) means polymerizable monomers having no acidic group with a solubility of less than 10 mass% in 25°C water.

Aside from hydrophobic monofunctional polymerizable monomers, other examples of the hydrophobic polymerizable monomer (A-2a) include crosslinkable polymerizable monomers such as aromatic bifunctional polymerizable monomers, aliphatic bifunctional polymerizable monomers, and tri- and higher-functional polymerizable monomers.

Examples of the hydrophobic monofunctional polymerizable monomers include methyl methacrylate, ethyl methacrylate, butyl methacrylate, benzyl methacrylate, tetrahydrofurfuryl methacrylate, isobornyl methacrylate, p-cumyl-phenoxyethylene glycol methacrylate (CMP-1E), 2-phenoxybenzyl methacrylate, stearyl methacrylate, dicyclopentanyl methacrylate, butoxydiethylene glycol methacrylate, methoxypolyethylene glycol methacrylate, and mixtures of these. Preferred among these are benzyl methacrylate, tetrahydrofurfuryl methacrylate, isobornyl methacrylate, CMP-1E, and mixtures of these.

Examples of the aromatic bifunctional polymerizable monomers include 2,2-bis((meth)acryloyloxyphenyl)propane, 2,2-bis[4-(2-hydroxy-3-(meth)acryloyloxypropoxy)phenyl]propane, 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, and 2,2-bis(4-(meth)acryloyloxyisopropoxyphenyl)propane. Preferred among these are 2,2-bis[4-(2-hydroxy-3-methacryloyloxypropoxy)phenyl]propane (commonly known as Bis-GMA), 2,2-bis(4-(meth)acryloyloxyethoxyphenyl)propane, 2,2-bis(4-methacryloyloxypolyethoxyphenyl)propane (with an average of 2.6 moles of ethoxy group added; commonly known as D-2.6E), 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, and 2,2-bis(4-(meth)acryloyloxypentaethoxyphenyl)propane.

Examples of the aliphatic bifunctional polymerizable monomers include glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, 1,3-butanediol di(meth)acrylate, 1,5-pentanediol di(meth)acrylate, 1,6-hexanediol di(meth)acrylate, 1,10-decanediol di(meth)acrylate, 1,2-bis(3-methacryloyloxy-2-hydroxypropoxy)ethane, 2,2,4-trimethylhexamethylenebis(2-carbamoyloxyethyl)di(meth)acrylate, N-methacryloyloxyethylacrylamide, and N-methacryloyloxypropylamide. Preferred among these are triethylene glycol diacrylate, triethylene glycol dimethacrylate (commonly known as 3G), neopentyl glycol di(meth)acrylate, 1,2-bis(3-methacryloyloxy-2-hydroxypropoxy)ethane, 2,2,4-trimethylhexamethylenebis(2-carbamoyloxyethyl)dimethacrylate (commonly known as UDMA), 1,10-decanediol dimethacrylate (commonly known as DD), and N-methacryloyloxyethylacrylamide (commonly known as MAEA).

Examples of the tri- and higher-functional polymerizable monomers include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol penta(meth)acrylate, N,N-(2,2,4-trimethylhexamethylene)bis[2-(aminocarboxy)propane-1,3-diol]tetra(meth)acr ylate, and 1,7-diacryloyloxy-2,2,6,6-tetra(meth)acryloyloxymethyl-4-oxaheptane. Preferred among these is N,N-(2,2,4-trimethylhexamethylene)bis[2-(aminocarboxy)propane-1,3-diol]tetramethacryl ate.

In view of mechanical strength and ease of handling, preferred among these hydrophobic polymerizable monomers (A-2a) are aromatic bifunctional polymerizable monomers, and aliphatic bifunctional polymerizable monomers. Preferred as aromatic bifunctional polymerizable monomers are Bis-GMA and D-2.6E. Preferred as aliphatic bifunctional polymerizable monomers are glycerol di(meth)acrylate, 3G, neopentyl glycol di(meth)acrylate, 1,6-hexanediol di(meth)acrylate, DD, 1,2-bis(3-methacryloyloxy-2-hydroxypropoxy)ethane, UDMA, and MAEA.

In view of providing favorable adhesive properties to tooth structure when used for the dental composition, the hydrophobic polymerizable monomers (A-2a) are more preferably Bis-GMA, D-2.6E, 3G, UDMA, DD, and MAEA, even more preferably Bis-GMA, D-2.6E, 3G, and MAEA.

The hydrophobic polymerizable monomers (A-2a) may be used alone, or two or more thereof may be used in combination. The content of the hydrophobic polymerizable monomer (A-2a) in a dental composition of the present invention is preferably 20 to 99 parts by mass, more preferably 40 to 95 parts by mass, even more preferably 60 to 95 parts by mass in 100 parts by mass of polymerizable monomer (A). With the content of hydrophobic polymerizable monomer (A-2a) falling in these ranges, the dental composition exhibits excellent wettability to tooth structure, providing desired adhesive properties, and the cured product exhibits desired mechanical strength.

### . Hydrophilic polymerizable monomer (A-2b) having no acidic group

It is preferable in a dental composition of the present invention that the polymerizable monomer (A) comprise a hydrophilic polymerizable monomer (A-2b) having no acidic group (hereinafter, also referred to simply as "hydrophilic polymerizable monomer (A-2b)"). The hydrophilic polymerizable monomer (A-2b) improves the wettability of the dental composition to tooth structure.

The hydrophilic polymerizable monomer (A-2b) is preferably a radical polymerizable monomer having no acidic group but having a polymerizable group. In view of ease of radical polymerization, the polymerizable group is preferably a (meth)acryloyloxy group and/or a (meth)acrylamide group. Here, hydrophilic polymerizable monomer (A-2b) means polymerizable monomers having no acidic group with a solubility of 10 mass% or more in 25°C water. The hydrophilic polymerizable monomer (A-2b) is preferably one having a solubility of 30 mass% or more in 25°C water, more preferably one capable of dissolving in water in any proportions at 25°C.

Preferably, the hydrophilic polymerizable monomers are those containing a hydrophilic group such as a hydroxyl group, an oxymethylene group, an oxyethylene group, an oxypropylene group, and an amide group.

Examples of the hydrophilic polymerizable monomer (A-2b) include hydrophilic monofunctional (meth)acrylate polymerizable monomers such as 2-hydroxyethyl (meth)acrylate, 3-hydroxypropyl (meth)acrylate, 2-hydroxypropyl (meth)acrylate, 1,3-dihydroxypropyl (meth)acrylate, 2,3-dihydroxypropyl (meth)acrylate, 2-((meth)acryloyloxy)ethyltrimethylammonium chloride, and polyethylene glycol di(meth)acrylate (with nine or more oxyethylene groups); and hydrophilic monofunctional (meth)acrylamide polymerizable monomers such as N-methylol(meth)acrylamide, N-hydroxyethyl(meth)acrylamide, N,N-bis(2-hydroxyethyl)(meth)acrylamide, N-methoxymethyl(meth)acrylamide, N-ethoxymethyl(meth)acrylamide, diacetone(meth)acrylamide, 4-(meth)acryloylmorpholine, N-trihydroxymethyl-N-methyl(meth)acrylamide, N,N-dimethylacrylamide, and N,N-diethylacrylamide.

In view of adhesive properties to tooth structure, preferred among these hydrophilic polymerizable monomers (A-2b) are 2-hydroxyethyl (meth)acrylate, 2,3-dihydroxypropyl (meth)acrylate, and hydrophilic monofunctional (meth)acrylamide polymerizable monomers, more preferably 2-hydroxyethyl (meth)acrylate, N,N-dimethylacrylamide, and N,N-diethylacrylamide. The hydrophilic polymerizable monomer (A-2b) may be incorporated alone, or two or more thereof may be used in combination.

The content of the hydrophilic polymerizable monomer (A-2b) in a dental composition of the present invention is preferably 0 to 50 parts by mass, more preferably 0 to 40 parts by mass, even more preferably 0 to 30 parts by mass in 100 parts by mass of polymerizable monomer (A). The content of hydrophilic polymerizable monomer (A-2b) may be 0 parts by mass in 100 parts by mass of polymerizable monomer (A). When the content of hydrophilic polymerizable monomer (A-2b) falls within these ranges, it produces an effect that enhances adhesive properties, and the cured product exhibits desired mechanical strength.

The content of polymerizable monomer (A-2) having no acidic group is preferably 50 to 99 parts by mass, more preferably 60 to 97 parts by mass, even more preferably 70 to 95 parts by mass in 100 parts by mass of polymerizable monomer (A).

### <Polymerization Initiator (B)>

The polymerization initiator (B) can be classified into a water-soluble photopolymerization initiator (B-1), a water-insoluble photopolymerization initiator (B-2), and a chemical polymerization initiator (B-3). The polymerization initiator (B) may be solely a water-soluble photopolymerization initiator (B-1), a water-insoluble photopolymerization initiator (B-2), or a chemical polymerization initiator (B-3), or may be a combination of a water-soluble photopolymerization initiator (B-1), a water-insoluble photopolymerization initiator (B-2), and a chemical polymerization initiator (B-3).

### · Water-soluble photopolymerization initiator (B-1)

With a water-soluble photopolymerization initiator (B-1), a high bond strength can be achieved by improving polymerization curability at the interface with hydrophilic tooth surfaces. The water-soluble photopolymerization initiator (B-1) has a solubility in 25°C water of 10 g/L or more, preferably 15 g/L or more, more preferably 20 g/L or more, even more preferably 25 g/L or more. By having a solubility of 10 g/L or more in 25°C water, the water-soluble photopolymerization initiator (B-1) can sufficiently dissolve in water within the tooth structure at the bonding interface, and more easily exhibits the polymerization promoting effect.

Examples of the water-soluble photopolymerization initiator (B-1) include water-soluble acylphosphine oxides; water-soluble thioxanthones; and α-hydroxyalkylacetophenones, for example, such as compounds having a (poly)ethylene glycol chain introduced into the hydroxyl group(s) of 1-[4-(2-hydroxyethoxy)-phenyl]-2-hydroxy-2-methyl-1-propan-1-one, compounds having a (poly)ethylene glycol chain introduced into the hydroxyl group and/or phenyl group of 1-hydroxycyclohexyl phenyl ketone, compounds having -OCH₂COO⁻Na⁺ introduced into the phenyl group of 1-hydroxycyclohexyl phenyl ketone, compounds having a (poly)ethylene glycol chain introduced into the hydroxyl group and/or phenyl group of 2-hydroxy-2-methyl-1-phenylpropan-1-one, and compounds having -OCH₂COO⁻Na⁺ introduced into the phenyl group of 2-hydroxy-2-methyl-1-phenylpropan-1-one. Other examples of the water-soluble photopolymerization initiator (B-1) include quaternary ammonium compounds prepared by quaternization of the amino group of α-aminoalkylphenones, such as 2-methyl-1-[4-(methylthio)phenyl]-2-morpholinopropan-1-one, and 2-benzyl-2-(dimethylamino)-1-[(4-morpholino)phenyl]-1-butanone.

The water-soluble thioxanthones may be any of, for example, 2-hydroxy-3-(9-oxo-9H-thioxanthen-4-yloxy)-N,N,N-trimethyl-1-propaneaminium chloride, 2-hydroxy-3-(1-methyl-9-oxo-9H-thioxanthen-4-yloxy)-N,N,N-trimethyl-1-propaneaminiu m chloride, 2-hydroxy-3-(9-oxo-9H-thioxanthen-2-yloxy)-N,N,N-trimethyl-1-propaneaminium chloride, 2-hydroxy-3-(3,4-dimethyl-9-oxo-9H-thioxanthen-2-yloxy)-N, N, N-trimethyl-1-propaneam i nium chloride, 2-hydroxy-3-(3,4-dimethyl-9H-thioxanthen-2-yloxy)-N,N,N-trimethyl-1-propaneaminium chloride, and 2-hydroxy-3-(1,3,4-trimethyl-9-oxo-9H-thioxanthen-2-yloxy)-N,N,N-trimethyl-1-propanea minium chloride.

Examples of the water-soluble acylphosphine oxides include acylphosphine oxides represented by the following general formula (4) or (5).

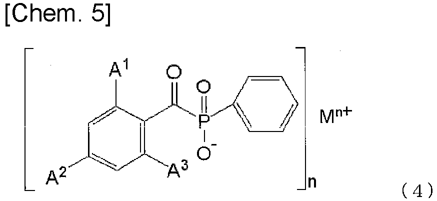

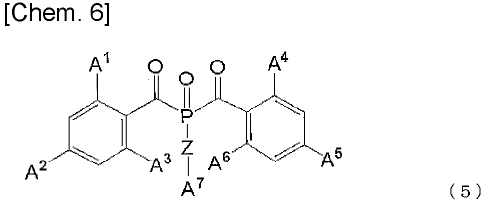

In the formulae, A¹, A², A³, A⁴, A⁵, and A⁶ are each independently a C₁ to C₄ linear or branched alkyl group or a halogen atom, M is a hydrogen ion, an alkali metal ion, an alkali earth metal ion, a magnesium ion, a pyridinium ion (the pyridine ring may have a substituent), or an ammonium ion represented by HN⁺A⁸A⁹A¹⁰ (where A⁸, A⁹, and A¹⁰ are each independently an organic group or a hydrogen atom), n is 1 or 2, Z is a C₁ to C₄ linear or branched alkylene group, and A⁷ represents -CH(CH₃)COO(C₂H₄O)ₚCH₃, where p represents an integer of 1 to 1,000.

The alkyl groups represented by A¹, A², A³, A⁴, A⁵, and A⁶ are not particularly limited, as long as they are C₁ to C₄ linear or branched alkyl groups. Examples include methyl groups, ethyl groups, n-propyl groups, isopropyl groups, n-butyl groups, isobutyl groups, sec-butyl groups, 2-methylpropyl groups, and tert-butyl groups. The alkyl groups represented by A¹, A², A³, A⁴, A⁵, and A⁶ are preferably C₁ to C₃ linear alkyl groups, more preferably methyl or ethyl groups, even more preferably methyl groups. Examples of the alkylene group represented by Z include methylene groups, ethylene groups, n-propylene groups, isopropylene groups, and n-butylene groups. The alkylene group represented by Z is preferably a C₁ to C₃ linear alkylene group, more preferably a methylene group or an ethylene group, even more preferably a methylene group.

Examples of the substituent of the pyridine ring when M is a pyridinium ion include halogen atoms (a fluorine atom, a chlorine atom, a bromine atom, an iodine atom), a carboxyl group, a C₂ to C₆ linear or branched acyl group, a C₁ to C₆ linear or branched alkyl group, and a C₁ to C₆ linear or branched alkoxy group. Preferably, M is an alkali metal ion, an alkali earth metal ion, a magnesium ion, a pyridinium ion (the pyridine ring may have a substituent), or an ammonium ion represented by HN⁺A⁸A⁹A¹⁰ (the symbols have the same meaning as above). Examples of the alkali metal ion include a lithium ion, a sodium ion, a potassium ion, a rubidium ion, and a cesium ion. Examples of the alkali earth metal ion include a calcium ion, a strontium ion, a barium ion, and a radium ion. The organic groups represented by A⁸, A⁹, and A¹⁰ may be the same groups exemplified above for the substituent of the pyridine ring (excluding a halogen atom).

In view of the storage stability and shade stability of the composition, particularly preferred are compounds in which A¹, A², A³, A⁴, A⁵, and A⁶ are all methyl groups. Examples of Mⁿ⁺ include Li⁺, Na⁺, K⁺, Ca²⁺, Mg²⁺, and ammonium ions derived from various amines. Examples of the amines include ammonia, trimethylamine, diethylamine, dimethylaniline, ethylenediamine, triethanolamine, N,N-dimethylaminomethacrylate, 4-(N,N-dimethylamino)benzoic acid and alkyl esters thereof, 4-(N,N-diethylamino)benzoic acid and alkyl esters thereof, and N,N-bis(2-hydroxyethyl)-p-toluidine.

In view of adhesive properties, p in A⁷ is 1 or more, preferably 2 or more, more preferably 3 or more, even more preferably 4 or more, and is 1,000 or less, preferably 100 or less, more preferably 75 or less, even more preferably 50 or less.

Particularly preferred among these water-soluble acylphosphine oxides are compounds represented by general formula (4) and in which Mⁿ⁺ is Li⁺, and compounds represented by general formula (5) and in which the moiety corresponding to the group represented by A⁷ is synthesized from polyethylene glycol methyl ether methacrylate having a molecular weight of 950. In these compounds, A¹, A², and A³ in general formula (4), and A¹, A², A³, A⁴, A⁵, and A⁶ in general formula (5) are as defined above.

The water-soluble acylphosphine oxides having such structures can be synthesized according to known methods, and some are available as commercially available products. For example, the methods disclosed in JP S57-197289 A and WO2014/095724 can be used for the synthesis of the water-soluble acylphosphine oxides. The water-soluble photopolymerization initiator (B-1) may be used alone, or two or more thereof may be used in combination.

The water-soluble photopolymerization initiator (B-1) may be dissolved in the dental composition, or may be dispersed in the form of a powder in the composition.

When the water-soluble photopolymerization initiator (B-1) is dispersed in the form of a powder in the composition, the average particle diameter is preferably 500 µm or less, more preferably 100 µm or less, even more preferably 50 µm or less because the water-soluble photopolymerization initiator (B-1) tends to settle when the average particle diameter of the powder is excessively large. The average particle diameter is preferably 0.01 µm or more because the specific surface area of the powder overly increases, and the amount of powder that can be dispersed in the composition decreases when the average particle diameter of the powder is excessively small. Taken together, the average particle diameter of water-soluble photopolymerization initiator (B-1) preferably ranges from 0.01 to 500 µm, more preferably 0.01 to 100 µm, even more preferably 0.01 to 50 µm.

The average particle diameter of a powder of individual water-soluble photopolymerization initiators (B-1) can be determined by taking an electron micrograph of at least 100 particles, and calculating the volume average particle diameter from the captured image after an image analysis with image-analyzing particle size distribution measurement software (Mac-View, manufactured by Mountech Co., Ltd.).

The shape of the water-soluble photopolymerization initiator (B-1) when it is dispersed in the form of a powder in the composition is not particularly limited, and may be any of various shapes, including, for example, spherical, stylus, plate-like, and crushed shapes. The water-soluble photopolymerization initiator (B-1) can be prepared using a known method such as pulverization, freeze drying, or reprecipitation. In view of the average particle diameter of the powder obtained, freeze drying and reprecipitation are preferred, and freeze drying is more preferred.

In view of curability and other properties of the dental composition obtained, the content of water-soluble photopolymerization initiator (B-1) is preferably 0.01 to 20 parts by mass relative to 100 parts by mass of the polymerizable monomer (A) in a dental composition of the present invention. In view of adhesive properties to tooth structure, the content is more preferably 0.05 to 10 parts by mass, even more preferably 0.1 to 5 parts by mass. When the content of water-soluble photopolymerization initiator (B-1) is 0.01 parts or more by mass, polymerization can sufficiently proceed at the bonding interface, providing desired adhesive properties. When the content of water-soluble photopolymerization initiator (B-1) is 20 parts or less by mass, it leads to sufficient adhesive properties.

### · Water-insoluble photopolymerization initiator (B-2)

In view of curability, a dental composition of the present invention preferably comprises a water-insoluble photopolymerization initiator (B-2) having a solubility of less than 10 g/L in 25°C water (hereinafter, also referred to as "water-insoluble photopolymerization initiator (B-2)"), other than the water-soluble photopolymerization initiator (B-1). The water-insoluble photopolymerization initiator (B-2) used in the present invention may be a known photopolymerization initiator. The water-insoluble photopolymerization initiator (B-2) may be incorporated alone, or two or more thereof may be incorporated in combination.

Examples of the water-insoluble photopolymerization initiator (B-2) include (bis)acylphosphine oxides (other than those exemplified for the water-soluble photopolymerization initiator (B-1)), thioxanthones, ketals, α-diketones, coumarins, anthraquinones, benzoin alkyl ether compounds, and α-aminoketone compounds.

Examples of acylphosphine oxides in the (bis)acylphosphine oxides include 2,4,6-trimethylbenzoyldiphenylphosphine oxide, 2,6-dimethoxybenzoyldiphenylphosphine oxide, 2,6-dichlorobenzoyldiphenylphosphine oxide, 2,4,6-trimethylbenzoylmethoxyphenylphosphine oxide, 2,4,6-trimethylbenzoylethoxyphenylphosphine oxide, 2,3,5,6-tetramethylbenzoyldiphenylphosphine oxide, and benzoyl di(2,6-dimethylphenyl)phosphonate. Examples of bisacylphosphine oxides include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, and bis(2,5,6-trimethylbenzoyl)-2,4,4-trimethylpentylphosphine oxide.

Examples of the thioxanthones include thioxanthone, and 2-chlorothioxanthen-9-one.

Examples of the ketals include benzyl dimethyl ketal, and benzyl diethyl ketal.

Examples of the α-diketones include diacetyl, benzyl, dl-camphorquinone, 2,3-pentadione, 2,3-octadione, 9,10-phenanthrenequinone, 4,4'-oxybenzyl, and acenaphthenequinone. Particularly preferred is dl-camphorquinone for its maximum absorption wavelength occurring in the visible light region.

Examples of the coumarins include compounds mentioned in JP H09-3109 A and JP H10-245525 A, including, for example, 3,3'-carbonylbis(7-diethylaminocoumarin), 3-(4-methoxybenzoyl)coumarin, 3-thienoylcoumarin, 3-benzoyl-5,7-dimethoxycoumarin, 3-benzoyl-7-methoxycoumarin, 3-benzoyl-6-methoxycoumarin, 3-benzoyl-8-methoxycoumarin, 3-benzoylcoumarin, 7-methoxy-3-(p-nitrobenzoyl)coumarin, 3-(p-nitrobenzoyl)coumarin, 3,5-carbonylbis(7-methoxycoumarin), 3-benzoyl-6-bromocoumarin, 3,3'-carbonylbiscoumarin, 3-benzoyl-7-dimethylaminocoumarin, 3-benzoylbenzo[f]coumarin, 3-carboxycoumarin, 3-carboxy-7-methoxycoumarin, 3-ethoxycarbonyl-6-methoxycoumarin, 3-ethoxycarbonyl-8-methoxycoumarin, 3-acetylbenzo[f]coumarin, 3-benzoyl-6-nitrocoumarin, 3-benzoyl-7-diethylaminocoumarin, 7-dimethylamino-3-(4-methoxybenzoyl)coumarin, 7-diethylamino-3-(4-methoxybenzoyl)coumarin, 7-diethylamino-3-(4-diethylamino)coumarin, 7-methoxy-3-(4-methoxybenzoyl)coumarin, 3-(4-nitrobenzoyl)benzo[f]coumarin, 3-(4-ethoxycinnamoyl)-7-methoxycoumarin, 3-(4-dimethylaminocinnamoyl)coumarin, 3-(4-diphenylaminocinnamoyl)coumarin, 3-[(3-dimethylbenzothiazol-2-ylidene)acetyl]coumarin, 3-[(1-methylnaphtho[1,2-d]thiazol-2-ylidene)acetyl]coumarin, 3,3'-carbonylbis(6-methoxycoumarin), 3,3'-carbonylbis(7-acetoxycoumarin), 3,3'-carbonylbis(7-dimethylaminocoumarin), 3-(2-benzothiazolyl)-7-(diethylamino)coumarin, 3-(2-benzothiazolyl)-7-(dibutylamino)coumarin, 3-(2-benzoimidazolyl)-7-(diethylamino)coumarin, 3-(2-benzothiazolyl)-7-(dioctylamino)coumarin, 3-acetyl-7-(dimethylamino)coumarin, 3,3'-carbonylbis(7-dibutylaminocoumarin), 3,3'-carbonyl-7-diethylaminocoumarin-7'-bis(butoxyethyl)aminocoumarin, 10-[3-[4-(dimethylamino)phenyl]-1-oxo-2-propenyl]-2,3,6,7-tetrahydro-1,1,7,7-tetramethy l-1H,5H,11H-[1]benzopyrrano[6,7,8-ij]quinolizin-11-one, and 10-(2-benzothiazolyl)-2,3,6,7-tetrahydro-1,1,7,7-tetramethyl-1H,5H,11H-[1]benzopyrrano [6,7,8-ij]quinolizin-11-one.

Particularly preferred among these coumarins are 3,3'-carbonylbis(7-diethylaminocoumarin), and 3,3'-carbonylbis(7-dibutylaminocoumarin).

Examples of the anthraquinones include anthraquinone, 1-chloroanthraquinone, 2-chloroanthraquinone, 1-bromoanthraquinone, 1,2-benzanthraquinone, 1-methylanthraquinone, 2-ethylanthraquinone, and 1-hydroxyanthraquinone.

Example of the benzoin alkyl ether compounds include benzoin methyl ether, benzoin ethyl ether, benzoin isopropyl ether, and benzoin isobutyl ether.

Examples of the α-aminoketone compounds include 2-methyl-1-[4-(methylthio)phenyl]-2-morpholinopropan-1-one.

Among these examples, the water-insoluble photopolymerization initiator (B-2) is preferably at least one selected from the group consisting of a (bis)acylphosphine oxide, an α-diketone, and a coumarin. In this way, a dental composition can be obtained that has excellent photocurability both in the visible light region and the near ultraviolet region, and that shows sufficient photocurability regardless of whether the light source used is a halogen lamp, a light emitting diode (LED), or a xenon lamp.

The content of water-insoluble photopolymerization initiator (B-2) is not particularly limited. However, in view of curability and other properties of the dental composition obtained, the content of water-insoluble photopolymerization initiator (B-2) is preferably 0.01 to 10 parts by mass, more preferably 0.05 to 7 parts by mass, even more preferably 0.1 to 5 parts by mass relative to 100 parts by mass of the polymerizable monomer (A) in a dental composition of the present invention. When the content of water-insoluble photopolymerization initiator (B-2) is 10 parts or less by mass, it leads to sufficient adhesive properties.

When the water-soluble photopolymerization initiator (B-1) and the water-insoluble photopolymerization initiator (B-2) are used in combination, the mass ratio (B-1):(B-2) of water-soluble photopolymerization initiator (B-1) and water-insoluble photopolymerization initiator (B-2) in the present invention is preferably 10:1 to 1:10, more preferably 7:1 to 1:7, even more preferably 5:1 to 1:5, most preferably 3:1 to 1:3. When the fraction of water-soluble photopolymerization initiator (B-1) in the mass ratio exceeds 10:1, the curability of the dental composition itself may decrease, and the dental composition may have difficulty in exhibiting high bond strength. When the fraction of water-insoluble photopolymerization initiator (B-2) in the mass ratio exceeds 1:10, the dental composition may have difficulty in exhibiting high bond strength as a result of insufficient promotion of polymerization at the bonding interface, though the curability of the dental composition itself can increase.

### · Chemical polymerization initiator (B-3)

A dental composition of the present invention may comprise a chemical polymerization initiator (B-3). Preferred for use are organic peroxides. The organic peroxides used as the chemical polymerization initiator are not particularly limited, and may be known organic peroxides. Typical examples of organic peroxides include ketone peroxides, hydroperoxides, diacyl peroxides, dialkyl peroxides, peroxyketals, peroxyesters, and peroxydicarbonates. Specific examples of these organic peroxides include those mentioned in WO2008/087977. The chemical polymerization initiator (B-3) may be incorporated alone, or two or more thereof may be incorporated in combination.

### <Filler (C)>

A dental composition of the present invention comprises a filler (C), in order to adjust the ease of handling, and to increase the mechanical strength of the cured product.

Examples of the filler (C) include a surface-treated filler for dental use (C-1) (described later), as well as fillers other than surface-treated filler for dental use (C-1), such as inorganic fillers, organic fillers, and organic-inorganic composite fillers. The filler (C) may be incorporated alone, or two or more thereof may be incorporated in combination.

A certain embodiment is, for example, a dental composition that comprises a polymerizable monomer (A), a polymerization initiator (B), and a filler (C), and in which the filler (C) comprises a surface-treated filler for dental use (C-1) surface-treated with a silane coupling agent (a) having a polymerizable group, and an end capping agent (b), along with a filler other than surface-treated filler for dental use (C-1), and the surface-treated filler for dental use (C-1) shows a dissolution rate of less than 100 ppm for metallic components in an acid resistance test.

Fillers other than surface-treated filler for dental use (C-1) are described first.

Examples of the materials of the organic fillers include polymethylmethacrylate, polyethylmethacrylate, a methyl methacrylate-ethyl methacrylate copolymer, crosslinked polymethylmethacrylate, crosslinked polyethylmethacrylate, polyamides, polyvinyl chloride, polystyrene, chloroprene rubber, nitrile rubber, an ethylene-vinyl acetate copolymer, a styrene-butadiene copolymer, an acrylonitrile-styrene copolymer, and an acrylonitrile-styrene-butadiene copolymer. These may be used alone, or two or more thereof may be used as a mixture. The shape of the organic fillers is not particularly limited, and their particle diameters may be appropriately selected for use. In view of considerations such as the ease of handling and mechanical strength of the dental composition obtained, the average particle diameter of the organic fillers is preferably 0.001 to 50 µm, more preferably 0.001 to 10 µm.

Examples of the materials of the inorganic fillers include various types of glass (containing silica as a main component, and, optionally, oxides of elements such as heavy metals, boron, and aluminum). Examples of such glass include glass powders of common compositions, such as fused silica, quartz, soda-lime-silica glass, E glass, C glass, and borosilicate glass (PYREX^{®} glass); and dental glass powders such as barium glass (GM27884 and 8235 manufactured by SCHOTT, and E-2000 and E-3000 manufactured by ESSTECH), strontium borosilicate glass (E-4000 manufactured by ESSTECH), lanthanum glass-ceramic (GM31684 manufactured by SCHOTT), and fluoroaluminosilicate glass (GM35429, G018-091, and G018-117 manufactured by SCHOTT). Other examples of inorganic filler materials include various types of ceramics, composite oxides (such as silica-titania, and silica-zirconia), silica-zirconia oxide aggregate fillers, diatomaceous earth, kaolin, clay minerals (such as montmorillonite), activated earth, synthetic zeolite, mica, calcium fluoride, ytterbium fluoride, yttrium fluoride, core-shell structured calcium fluoride with silica coating, core-shell structured ytterbium fluoride with silica coating, core-shell structured yttrium fluoride with silica coating, calcium phosphate, barium sulfate, zirconium dioxide, titanium dioxide, hydroxyapatite, core-shell structured calcium phosphate with silica coating, core-shell structured barium sulfate with silica coating, core-shell structured zirconium dioxide with silica coating, core-shell structured titanium dioxide with silica coating, and core-shell structured hydroxyapatite with silica coating. In view of strength and other properties, preferred among these are various types of glass, composite oxides (such as silica-titania, and silica-zirconia), silica-zirconia oxide aggregate fillers, core-shell structured calcium fluoride with silica coating, core-shell structured ytterbium fluoride with silica coating, core-shell structured yttrium fluoride with silica coating, core-shell structured calcium phosphate with silica coating, core-shell structured barium sulfate with silica coating, core-shell structured zirconium dioxide with silica coating, core-shell structured titanium dioxide with silica coating, and core-shell structured hydroxyapatite with silica coating. These may be used alone, or two or more thereof may be used in combination. In the present invention, the average particle diameter of filler means the average particle diameter before surface treatment when the filler is subjected to surface treatment.

Concerning the shape of inorganic fillers, the inorganic fillers may be, for example, irregularly shaped fillers or spherical fillers. In view of enhancing the mechanical strength of the cured product of the dental composition, the inorganic fillers are preferably spherical fillers. The spherical fillers used in the present invention are particles that appear round in shape when observed in a unit field of an electron micrograph, and that have an average uniformity of 0.6 or more as calculated by dividing the diameter of a particle perpendicular to its maximum diameter by the maximum diameter. The fillers have an average particle diameter of preferably 0.05 to 50 µm, more preferably 0.1 to 30 µm, even more preferably 0.5 µm to 30 µm. When the average particle diameter is less than 0.05 µm, the mechanical strength may decrease as a result of a decrease in the filling rate of the filler in the dental composition. When the average particle diameter is more than 50 µm, it may not be possible to obtain a cured product having high mechanical strength as a result of a decrease in the surface area of the filler.

In certain embodiments, it is preferable to comprise aggregate fillers as inorganic fillers. Aggregate fillers are fillers with aggregated primary particles forming secondary particles, characterized by the ability to produce high mechanical strength and achieve superior polishability (ease of achieving the desired gloss with short polishing time).

The aggregate fillers may be used alone, or two or more thereof may be used in combination.

The aggregate fillers are preferably aggregates of metal oxide primary particles.

The metal oxides may be composite oxides. Examples of the composite oxides include silica-zirconia, and silica-titania.

In view of properties such as the polishability of the dental composition obtained, the metal oxides are preferably silica-zirconia oxides.

In view of properties such as the ease of handling and mechanical strength of the dental composition obtained, the secondary particles of aggregate fillers have an average particle diameter of 1 to 20 µm, preferably 1.5 to 15 µm, more preferably 2 to 12.5 µm, even more preferably 3 to 10 µm.

In view of properties such as the polishability of the dental composition obtained, the primary particles of aggregate fillers have an average particle diameter of preferably 30 to 500 nm, more preferably 40 to 400 nm, even more preferably 50 to 300 nm, particularly preferably 60 to 200 nm.

The aggregate fillers may be commercially available products.

Examples of the commercially available products include silica-zirconia oxide aggregate fillers SG-SZ200G151CMP8, SG-SZ50G151CMP8, and SG-SZ200G154CMP8 (manufactured by Sukgyung AT).

The organic-inorganic composite fillers usable in the present invention are fillers obtained by adding a polymerizable monomer to the inorganic filler, polymerizing the mixture in paste form, and pulverizing the polymerized filler. The organic-inorganic composite fillers may be, for example, TMPT fillers (a mixture of trimethylolpropane methacrylate and silica filler pulverized after polymerization). The shape of the organic-inorganic composite fillers is not particularly limited, and their particle diameters may be appropriately selected for use. In view of properties such as the ease of handling and mechanical strength of the composition obtained, the organic-inorganic composite fillers have an average particle diameter of preferably 0.001 to 50 µm, more preferably 0.001 to 10 µm.

In this specification, the average particle diameter of filler can be determined using a laser diffraction scattering method or by observing particles with an electron microscope. Specifically, a laser diffraction scattering method is more convenient for the measurement of particles having a particle diameter of 0.1 µm or more, whereas electron microscopy is a more convenient method of particle size measurement for ultrafine particles of less than 0.1 µm. Here, 0.1 µm is a measured value by a laser diffraction scattering method.

As a specific example of a laser diffraction scattering method, the particle size may be measured by volume using, for example, a laser diffraction particle size distribution analyzer (SALD-2300, manufactured by Shimadzu Corporation) with a 0.2% sodium hexametaphosphate aqueous solution used as dispersion medium.

In electron microscopy, for example, particles may be photographed with an electron microscope (Model S-4000, manufactured by Hitachi, Ltd.), and the size of particles (at least 200 particles) observed in a unit field of the micrograph may be measured using image-analyzing particle size distribution measurement software (Macview, manufactured by Mountech Co., Ltd.). Here, the particle diameter is determined as an arithmetic mean value of the maximum and minimum lengths of particles, and the average primary particle diameter is calculated from the number of particles and the particle diameter.

In a dental composition of the present invention, it is preferable to use a mixture or a combination of two or more fillers of different materials, different particle size distributions, and different forms. By combining two or more types of fillers, the fillers can interact with the polymerizable monomer or with the other fillers at an increased number of points, in addition to densely packing themselves. Depending on the type of filler, it is also possible to control paste flowability in the presence or absence of a shear force. Different types of fillers may be incorporated within fillers of different particle sizes. Particles other than fillers may be unintentionally present as impurities, as long as it does not hinder the effectiveness of the present invention.

In order to adjust the flowability of the dental composition, the filler may be used after an optional surface treatment with a known surface treatment agent such as a silane coupling agent. Examples of such surface treatment agents include vinyltrimethoxysilane, vinyltriethoxysilane, vinyltrichlorosilane, vinyltri(β-methoxyethoxy)silane, 3-methacryloyloxypropyltrimethoxysilane, 8-methacryloyloxyoctyltrimethoxysilane, 11-methacryloyloxyundecyltrimethoxysilane, 3-glycidoxypropyltrimethoxysilane, 3-mercaptopropyltrimethoxysilane, and 3-aminopropyltriethoxysilane.

### · Surface-treated filler for dental use (C-1) surface-treated with silane coupling agent (a) having a polymerizable group, and end capping agent (b)

A dental composition of the present invention comprises a surface-treated filler for dental use (C-1) as one of the filler (C). The surface-treated filler for dental use (C-1) is a filler surface-treated with a silane coupling agent (a) having a polymerizable group (hereinafter, also referred to simply as "silane coupling agent (a)"), and an end capping agent (b) (hereinafter, this filler is also referred to simply as "surface-treated filler for dental use (C-1)"). By comprising the surface-treated filler for dental use (C-1), the dental composition can exhibit excellent storage stability even under severe conditions (for example, 60°C for 4 weeks), and show sufficient mechanical strength in its cured product. The surface-treated filler for dental use (C-1) shows a dissolution rate of less than 100 ppm for metallic components in an acid resistance test. The dissolution rate for metallic components is preferably 95 ppm or less, more preferably 90 ppm or less, even more preferably 70 ppm or less, particularly preferably 50 ppm or less. The measurement method of the dissolution rate for metallic components in an acid resistance test is as described in the EXAMPLES section below.

The surface-treated filler for dental use (C-1) is preferably one in which a structure derived from a silane coupling agent (a), and a structure derived from an end capping agent (b) are present on its surface. The surface-treated filler for dental use (C-1) is preferably one in which a structure derived from a silane coupling agent (a) is chemically attached to its surface. The surface-treated filler for dental use (C-1) is preferably one in which a structure derived from an end capping agent (b) is chemically attached to its surface. Preferably, the surface-treated filler for dental use (C-1) has a structure in which a structure derived from a silane coupling agent (a), and a structure derived from an end capping agent (b) are chemically attached to each other.

### · Silane coupling agent (a) having a polymerizable group

The surface-treated filler for dental use (C-1) is surface-treated with a silane coupling agent (a) having a polymerizable group, along with other surface treatment agents. The silane coupling agent (a) is not particularly limited, and may be a known silane coupling agent. Preferred for use are silane coupling agents represented by general formula (6).

Y-SiRₚX₍₃₋ₚ₎ (6)

wherein Y represents a polymerizable group, or a monovalent organic group having a polymerizable group, R represents a group selected from the group consisting of an alkyl group, an aryl group, and an aralkyl group, X represents a hydroxyl group or a hydrolyzable group, and p represents an integer of 0, 1, or 2. The plurality of R may be the same or different from each other. The plurality of X may be the same or different from each other.

The polymerizable group represented by Y is not particularly limited. Examples include (meth)acryloyl groups, vinyl groups, mercapto groups, (meth)allyl groups, and epoxy groups. In view of considerations such as mechanical strength, preferred are (meth)acryloyl groups, more preferably methacryloyl groups. The polymerizable group may be bound to a monovalent organic group either directly or via a divalent group containing a heteroatom such as an oxygen atom or nitrogen atom. For example, the (meth)acryloyl group may form a (meth)acryloyloxy group or an (meth)acrylamide group.

The number of polymerizable groups represented by Y is not particularly limited, and is preferably 1 to 4, more preferably 1 or 2, even more preferably 1. When Y represents a plurality of polymerizable groups, the polymerizable groups may be the same or different from each other.

Y may be formed solely by the polymerizable group, or may be a group in which the functional group is directly bound to an organic group, or indirectly via, for example, a divalent group having a heteroatom such as an oxygen atom or nitrogen atom. The organic group is not particularly limited, and is, for example, an alkyl group having 1 to 20 carbon atoms, an aryl group having 6 to 18 carbon atoms, or an aralkyl group having 7 to 26 carbon atoms. For considerations such as further improvement of adhesive properties to both dental restoration materials and tooth structures, preferred among these are alkyl groups having 1 to 20 carbon atoms, more preferably alkyl groups having 1 to 12 carbon atoms, even more preferably alkyl groups having 3 to 11 carbon atoms.

Examples of alkyl groups having 3 to 11 carbon atoms include n-propyl groups, isopropyl groups, n-butyl groups, n-pentyl groups, n-octyl groups, and n-undecyl groups. Preferred are n-propyl groups, n-pentyl groups, n-octyl groups, and n-undecyl groups, more preferably n-propyl groups, n-octyl groups, and n-undecyl groups.

Specific examples of Y include (meth)acryloyloxymethyl groups, 3-(meth)acryloyloxypropyl groups, 3-(meth)acrylamidepropyl groups, vinyl groups, (meth)allyl groups, and 3-glycidoxypropyl groups. Preferred are (meth)acryloyloxymethyl groups, 3-(meth)acryloyloxypropyl groups, 3-(meth)acryloyloxyoctyl groups, and 3-(meth)acryloyloxyundecyl groups, more preferably 3-(meth)acryloyloxypropyl groups, 8-(meth)acryloyloxyoctyl groups, and 11-(meth)acryloyloxyundecyl groups.

The alkyl group represented by R is not particularly limited, and may be, for example, an alkyl group having 1 to 5 carbon atoms. Specific examples include methyl groups, ethyl groups, n-propyl groups, isopropyl groups, n-butyl groups, and n-pentyl groups.

The aryl group represented by R is not particularly limited, and may be, for example, an aryl group having 6 to 10 carbon atoms. Specific examples include phenyl groups and naphthyl groups.

The aralkyl group represented by R is not particularly limited, and may be, for example, an aralkyl group having 7 to 12 carbon atoms. Specific examples include benzyl groups.

In view of mechanical strength and other properties, R is preferably an alkyl group, more preferably an alkyl group having 1 to 5 carbon atoms, even more preferably a methyl group.

The hydrolyzable group represented by X may be a group that, through hydrolysis, has the capability to form a silanol group with the silicon atom to which it is attached. Examples include alkoxy groups, acyloxy groups, siloxy groups, and halogen atoms.

The alkoxy groups are not particularly limited, and may be, for example, alkoxy groups having 1 to 5 carbon atoms. Specific examples include methoxy groups, ethoxy groups, n-propoxy groups, isopropoxy groups, n-butoxy groups, and n-pentyloxy groups.

The acyloxy groups are not particularly limited, and may be, for example, acyloxy groups having 1 to 5 carbon atoms. Specific examples include formyloxy groups, acetoxy groups, n-propionyloxy groups, isopropionyloxy groups, n-butanoyloxy groups, and n-pentanoyloxy groups.

The siloxy groups are not particularly limited, and may be, for example, trimethylsiloxy groups.

The halogen atoms are not particularly limited, and may be, for example, a chlorine atom or a bromine atom.

Preferably, X is an alkoxy group, more preferably an alkoxy group having 1 to 5 carbon atoms, even more preferably a methoxy group or an ethoxy group.

The symbol p represents an integer of 0, 1, or 2. In view of properties such as the mechanical strength of the cured product, p is preferably 0 or 1. When p is 0 or 1, the multiple X may be the same or different from each other. When p is 2, the multiple R may be the same or different from each other.

Specific examples of the silane coupling agent (a) include:
silane coupling agents containing a (meth)acryloyl group, such as (meth)acryloyloxymethyltrimethoxysilane, 2-(meth)acryloyloxyethyltrimethoxysilane, 3-(meth)acryloyloxypropyltrimethoxysilane, 4-(meth)acryloyloxybutyltrimethoxysilane, 5-(meth)acryloyloxypentyltrimethoxysilane, 6-(meth)acryloyloxyhexyltrimethoxysilane, 7-(meth)acryloyloxyheptyltrimethoxysilane, 8-(meth)acryloyloxyoctyltrimethoxysilane, 9-(meth)acryloyloxynonyltrimethoxysilane, 10-(meth)acryloyloxydecyltrimethoxysilane, 11 -(meth)acryloyloxyundecyltrimethoxysilane, 11-(meth)acryloyloxyundecyldichloromethylsilane, 11-(meth)acryloyloxyundecyltrichlorosilane, 11-(meth)acryloyloxyundecyldimethoxymethylsilane, 12-(meth)acryloyloxydodecyltrimethoxysilane, 13-(meth)acryloyloxytridecyltrimethoxysilane, 3-(meth)acryloyloxypropylmethyldimethoxysilane, 3-(meth)acryloyloxypropylmethyldiethoxysilane, 3-(meth)acryloyloxypropylmethyldiisopropoxysilane, 3-(meth)acryloyloxypropylmethyldimethylsiloxysilane, 3-(meth)acryloyloxypropylmethyldihexyloxysilane, (meth)acryloyloxy-2-(2-vinyloxyethoxy)ethylmethyldimethoxysilane, 6-(meth)acryloyloxyhexylmethyldimethoxysilane, (meth)acryloyloxy-p-phenylethylmethyldimethoxysilane, 6-(meth)acryloyloxyhexylmethyldiethoxysilane, 10-(meth)acryloyloxydecylmethyldimethoxysilane, 11-(meth)acryloyloxyundecylmethyldimethoxysilane, 11-(meth)acryloyloxyundecylmethyldiethoxysilane, 11-(meth)acryloyloxyundecylmethyldihexyloxysilane, 20-(meth)acryloyloxyeicosylmethyldimethoxysilane, 3-(meth)acryloyloxypropylphenyldimethoxysilane, 3-(meth)acryloyloxypropylmethyldichlorosilane, 11-(meth)acryloyloxyundecylmethyldichlorosilane, 11-(meth)acryloyloxyundecylethyldichlorosilane, 3-(meth)acryloyloxypropyldimethylmonomethoxysilane, 3-(meth)acryloyloxypropyldimethylmonoethoxysilane, 3-(meth)acryloyloxypropyldimethylmonoisopropoxysilane, 3-(meth)acryloyloxypropyldimethylmonotrimethylsiloxysilane, 3-(meth)acryloyloxypropyldimethylmonohexyloxysilane, (meth)acryloyloxy-2-(2-vinyloxyethoxy)ethyldimethylmonomethoxysilane, 6-(meth)acryloyloxyhexyldimethylmonomethoxysilane, (meth)acryloyloxy-p-phenylethyldimethylmonomethoxysilane, 6-(meth)acryloyloxyhexyldimethylmonoethoxysilane, 10-(meth)acryloyloxydecyldimethylmonomethoxysilane, 11-(meth)acryloyloxyundecyldimethylmonomethoxysilane, 11-(meth)acryloyloxyundecyldimethylmonoethoxysilane, 11-(meth)acryloyloxyundecyldimethylmonohexyloxysilane, 20-(meth)acryloyloxyeicosyldimethylmonomethoxysilane, 3-(meth)acryloyloxypropyldiphenylmonomethoxysilane, 3-(meth)acryloyloxypropyldimethylmonochlorosilane, 11-(meth)acryloyloxyundecyldimethylmonochlorosilane, and 11-(meth)acryloyloxyundecyldiethylmonochlorosilane;
silane coupling agents containing a vinyl group, such as vinylmethyldimethoxysilane, vinylmethyldiethoxysilane, vinylmethyldichlorosilane, vinylmethyldiacetoxysilane, vinylmethyldi(2-methoxyethoxy)silane, vinyldimethylmonomethoxysilane, vinyldimethylmonoethoxysilane, vinyldimethylmonochlorosilane, vinyldimethylmonoacetoxysilane, and vinyldimethylmono(2-methoxyethoxy)silane;
silane coupling agents containing an epoxy group, such as 3-glycidoxypropylmethyldimethoxysilane, 3-glycidoxypropylmethyldiethoxysilane, 3-glycidoxypropyldimethylmonomethoxysilane, and 3-glycidoxypropyldimethylmonoethoxysilane; and
silane coupling agents containing an allyl group, such as allylmethyldiethoxysilane, and allyldimethylmonoethoxysilane.

The silane coupling agent (a) may be a hydrolysate and/or condensate of these. The silane coupling agent (a) may be used alone, or two or more thereof may be used in combination. In view of considerations such as mechanical strength, preferred among these are 3-methacryloyloxypropyltrimethoxysilane, 8-(meth)acryloyloxyoctyltrimethoxysilane, 11-(meth)acryloyloxyundecyltrimethoxysilane, and hydrolysates of these.

### · End capping agent (b)

The surface-treated filler for dental use (C-1) is surface-treated with an end capping agent (b), along with the silane coupling agent (a). Preferred as end capping agent (b) are compounds having a structure represented by the following general formula (1) or (2), and a boiling point of 180°C or less. 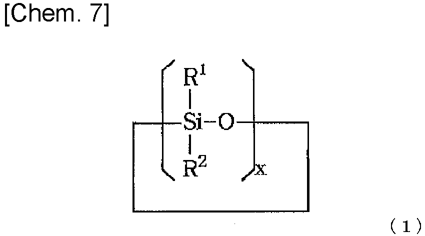 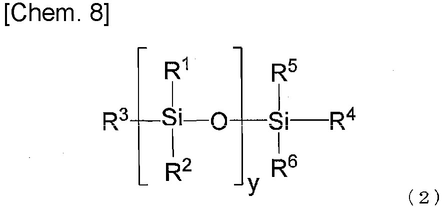 where R¹ to R⁶ each independently represent a hydrogen atom, a hydroxyl group, an alkyl group having 1 to 9 carbon atoms, or an alkoxy group having 1 to 4 carbon atoms. The symbol x represents an integer of 3 to 10, and the symbol y represents an integer of 1 to 100.

Because a reaction temperature of 180°C or less is necessary to enable reaction without deactivating the silane coupling agent (a) having a polymerizable group, the end capping agent (b) requires a boiling point of 180°C or less. The boiling point is preferably 170°C or less, more preferably 160°C or less, even more preferably 155°C or less, most preferably 150°C or less. In view of ease of handling, the end capping agent (b) has an boiling point of preferably 50°C or more, more preferably 60°C or more, even more preferably 70°C or more, most preferably 80°C or more.

Through surface treatment with the end capping agent (b), the surface-treated filler for dental use (C-1) can maintain the activity of the polymerizable groups present in the silane coupling agent (a), thereby demonstrating superior acid resistance and preventing reaction with polymerizable monomers having an acidic group. Consequently, the dental composition comprising polymerizable monomers having an acidic group exhibits excellent storage stability even under severe conditions (for example, 60°C for 4 weeks), even when the dental composition comprises an X-ray opacity filler. This is advantageous in not limiting the type of filler (C). Because the deactivation of the polymerizable groups present in the silane coupling agent (a) can be prevented through surface treatment with the end capping agent (b), and there is no situation where the other components impede this effect of the end capping agent (b), a dental composition of the present invention can exhibit excellent storage stability even under severe conditions, and show sufficient mechanical strength in its cured product, even when used in applications such as dental cements, dental bonding materials (for example, orthodontic bonding materials), dental composite resins (for example, such as self-adhesive dental composite resins), pit and fissure sealants, loose tooth fixing materials, and dental abutment construction materials.

In view of reactivity and boiling point, the alkyl groups represented by R¹ to R⁶ have preferably 1 to 5 carbon atoms, more preferably 1 to 4 carbon atoms, even more preferably 1 to 2 carbon atoms, most preferably 1 carbon atom. The symbol x is preferably 3 to 6, more preferably 3 to 5, even more preferably 3 to 4, most preferably 3. The symbol y is preferably 1 to 10, more preferably 1 to 4, even more preferably 1 to 2, most preferably 1. The symbol m is preferably 1 to 5, more preferably 1 to 4, even more preferably 1 to 2, most preferably 1.

C1 to C9 alkyl groups represented by R¹ to R⁶ may be linear or branched. Examples include methyl groups, ethyl groups, n-propyl groups, isopropyl groups, n-butyl groups, isobutyl groups, tert-butyl groups, n-pentyl groups, isopentyl groups, sec-pentyl groups, neopentyl groups, n-hexyl groups, n-heptyl groups, n-octyl groups, and n-nonyl groups.

A certain preferred embodiment is, for example, a dental composition in which the end capping agent (b) is a compound having the cyclic siloxane structure represented by general formula (1), and a boiling point of 180°C or less.

Another certain preferred embodiment is, for example, a dental composition in which the end capping agent (b) is a compound having the structure represented by general formula (2), and a boiling point of 180°C or less.

In a certain embodiment, it is preferable in the structure represented by general formula (2) that R¹ to R⁶ be each independently a hydrogen atom or an alkyl group having 1 to 4 carbon atoms, more preferably a hydrogen atom or an alkyl group having 1 to 3 carbon atoms, even more preferably a hydrogen atom, a methyl group, or an ethyl group, most preferably a hydrogen atom or a methyl group.

In another certain embodiment, the structure represented by general formula (2) has preferably 2 or more SiH groups, more preferably 3 or more SiH groups, even more preferably 4 or more SiH groups, most preferably 5 or more SiH groups when R¹ to R⁶ are alkyl groups having 1 to 9 carbon atoms, or alkoxy groups having 1 to 4 carbon atoms. It is also preferable that the structure represented by general formula (2) have 2 or more alkoxy groups directly attached to the silicon atoms, when R¹ to R⁶ include an alkoxy group.

In yet another certain embodiment, R¹ to R⁶ are preferably alkyl groups having 1 to 4 carbon atoms, or alkoxy groups having 1 to 4 carbon atoms, more preferably alkyl groups having 1 to 3 carbon atoms, or alkoxy groups having 1 to 3 carbon atoms, even more preferably alkyl groups having 1 to 2 carbon atoms, or alkoxy groups having 1 to 2 carbon atoms, most preferably alkoxy groups having 1 carbon atom, or alkyl groups having 1 carbon atom. The structure represented by general formula (2) has preferably 3 or more alkoxy groups, more preferably 4 or more alkoxy groups, even more preferably 5 or more alkoxy groups, most preferably 6 or more alkoxy groups when at least one of R¹ to R⁶ is an alkoxy group.

Specific examples of the end capping agent (b) include hexamethylcyclotrisiloxane (boiling point: 134°C), octamethylcyclotetrasiloxane (boiling point: 175°C), 1,1,3,3-tetramethyldisiloxane (boiling point: 71°C), 1,1,3,3,5,5-hexamethyltrisiloxane (boiling point: 128°C), and 1,3-dimethoxytetramethyldisiloxane. The end capping agent (b) may be used alone, or two or more thereof may be used in combination. In view of considerations such as reactivity, preferred among these is hexamethylcyclotrisiloxane.

The fillers (fillers before surface treatment) used for the surface-treated filler for dental use (C-1) are not particularly limited. Examples include the inorganic fillers and organic-inorganic composite fillers described as fillers (C). Preferred are inorganic fillers.

Specifically, preferred examples of inorganic filler materials include various types of glass (containing silica as a main component, and, optionally, oxides of elements such as heavy metals, boron, and aluminum). Examples of such glass include glass powders of common compositions, such as fused silica, quartz, soda-lime-silica glass, E glass, C glass, and borosilicate glass (PYREX^{®} glass), and dental glass powders such as barium glass (GM27884 and 8235 manufactured by SCHOTT, and E-2000 and E-3000 manufactured by ESSTECH), strontium borosilicate glass (E-4000 manufactured by ESSTECH), lanthanum glass-ceramic (GM31684 manufactured by SCHOTT), and fluoroaluminosilicate glass (GM35429, G018-091, and G018-117 manufactured by SCHOTT). Other preferred examples of inorganic filler materials include composite oxides (such as silica-titania, and silica-zirconia), silica-zirconia oxide aggregate fillers, ytterbium fluoride, core-shell structured ytterbium fluoride with silica coating, core-shell structured yttrium fluoride with silica coating, barium sulfate, zirconium dioxide, core-shell structured barium sulfate with silica coating, and core-shell structured zirconium dioxide with silica coating. In view of exhibiting X-ray opacity, preferred among these are X-ray opacity fillers such as barium glass, lanthanum glass-ceramic, fluoroaluminosilicate glass, composite oxides (such as silica-titania, and silica-zirconia), ytterbium fluoride, core-shell structured ytterbium fluoride with silica coating, core-shell structured yttrium fluoride with silica coating, barium sulfate, zirconium dioxide, core-shell structured barium sulfate with silica coating, and core-shell structured zirconium dioxide with silica coating.

It is to be noted that "X-ray opacity" denotes the characteristic of substances that do not allow X-rays to pass through, resulting in white appearances in areas with no X-ray passage in X-ray images. In this specification, "X-ray opacity" signifies the capacity to distinguish between natural tooth structure and hardened dental compositions using traditional techniques with standard dental X-ray equipment. Because dental compositions exhibit X-ray opacity when they contain X-ray opacity fillers, such dental compositions are more preferably used for diagnosing tooth conditions using X-rays compared to dental compositions that do not contain X-ray opacity fillers.

In view of considerations such as mechanical strength, preferred are barium glass, strontium borosilicate glass, lanthanum glass-ceramic, fluoroaluminosilicate glass, composite oxides (such as silica-zirconia), core-shell structured ytterbium fluoride with silica coating, and silica-zirconia oxide aggregate fillers, more preferably barium glass, strontium borosilicate glass, lanthanum glass-ceramic, fluoroaluminosilicate glass, core-shell structured ytterbium fluoride with silica coating, and silica-zirconia oxide aggregate fillers. These may be used alone, or two or more thereof may be used in combination.

The surface-treated filler for dental use (C-1) results from a reaction (dehydrocondensation) of the silane coupling agent (a) having a polymerizable group and the end capping agent (b) with the hydroxyl groups on the filler surface. The term "surface treatment" is used to refer to such a reaction. The process of such surface treatment is described as "surface treatment process".

Through surface treatment with the end capping agent (b), the hydroxyl groups present on the filler surface and that did not react with the silane coupling agent (a) (unreacted hydroxyl groups) undergo a reaction with the end capping agent (b).

The sequence of surface treatment to obtain the surface-treated filler for dental use (C-1) is not particularly limited. For example, the surface treatment of the filler may involve adding the silane coupling agent (a) and end capping agent (b) sequentially or simultaneously. For example, the sequence of surface treatment may involve reacting the filler with the silane coupling agent (a) first, followed by a specific end capping agent (b). Alternatively, the filler may undergo a reaction with the end capping agent (b) first, followed by silane coupling agent (a). In view of greater enhancement of surface coverage by silane coupling agent (a) and end capping agent (b), the sequence of surface treatment preferably involves reacting the filler with the silane coupling agent (a) first, followed by end capping agent (b).

A certain embodiment is, for example, a surface-treated filler for dental use (C-1) that results from surface treatment with the end capping agent (b) after surface treatment with the silane coupling agent (a).

The surface treatment method for obtaining the surface-treated filler for dental use (C-1) is not particularly limited, as long as it involves binding the silane coupling agent (a) to the filler surface through a dehydration polycondensation reaction, and binding the end capping agent (b) to the filler surface. As an example of the reaction with the silane coupling agent (a), the method may involve stirring the filler in a mixing vessel while spraying a solution of a surface treatment agent containing silane coupling agent (a) (preferably, only the silane coupling agent (a)) diluted in a solvent, and heating and drying the mixture for a certain duration in the vessel while continuously stirring it, or the method may involve stirring and mixing the filler with a surface treatment agent containing silane coupling agent (a) (preferably, only the silane coupling agent (a)) in a solvent, followed by heating and drying. The solvent is not particularly limited, and may be an alcohol-based solvent (such as methanol, ethanol, or isopropanol), water, or a mixed solvent of these. In the surface treatment with the silane coupling agent (a), the solvent is preferably one containing an acid, for example, acetic acid, (for example, an aqueous solution of acetic acid) because the reaction (hydrolysis reaction and dehydration condensation reaction) accelerates in an acidic environment. The heating temperature is not particularly limited, and may be about 30 to 90°C. As an example of the reaction with the end capping agent (b), the method may involve vaporizing a surface treatment agent containing the end capping agent (b) (preferably, only the end capping agent (b)) to allow for a reaction with the filler in the gas phase. The reaction temperature is not particularly limited, and may be appropriately altered according to the boiling point of the end capping agent (b). The preferred reaction temperature is 130 to 180°C.

In view of increasing reaction sites, and removing polyvalent metals to improve storage stability, it is preferable in the production of surface-treated filler for dental use (C-1) to conduct a treatment process with acid before surface treatment (hereinafter, also referred to simply as "acid treatment process"). The acid allows for the removal of polyvalent metals on the filler surface, inhibiting the adsorption of the polymerizable monomer (A-1) having an acidic group.

The acids used for the acid treatment process are not particularly limited, and may be organic acids or inorganic acids. Examples of the inorganic acids include sulfuric acid, hydrochloric acid, nitric acid, and sulfonic acid. Examples of the organic acids include formic acid, acetic acid, propionic acid, oxalic acid, citric acid, and tartaric acid. For considerations such as ease of removal after the process, preferred are inorganic acids, more preferably hydrochloric acid.

The method of acid treatment is not particularly limited, as long as there is contact between the filler and the acid. For example, the preferred method is to stir the filler in an acidic aqueous solution.

The concentration of the acidic aqueous solution used for the acid treatment process is preferably 1 to 20 mass%, more preferably 1 to 10 mass%. Preferably, the acidic aqueous solution with this concentration is used in an amount of 100 to 1,000 parts by mass relative to 100 parts by mass of the filler.

The acid treatment temperature in the acid treatment process is not particularly limited, and is preferably 10 to 60°C. The processing time can be appropriately set according to the acid treatment temperature, and the type and amount of acid used. The acid treatment process may be repeated.

For the filler treated with acid (hereinafter, also referred to simply as "acid-treated material"), it is possible to conduct a process of washing the acid-treated material, and/or adjusting the pH of the acid-treated material itself or the solution containing it to near neutral levels. The process of washing the acid-treated material (hereinafter, also referred to simply as "washing process") is a process that washes away any residual acid from the surface of the acid-treated material with water. The washing process may be repeated until all remaining acid is removed. When there is a notable amount of remaining acid, it may lead to an inadequate surface treatment in the surface treatment process. The pH of the acid-treated material or the solution containing it can be brought to near neutral using methods such as adding a pH adjuster (such as sodium bicarbonate), or repeating water washing and filtration to approach neutrality.

When the surface treatment process to produce the surface-treated filler for dental use (C-1) involves reacting the filler with the silane coupling agent (a) first, followed by the end capping agent (b), it is presumed that unreacted reactive groups (hydroxyl groups or hydrolyzable groups) may still be present on the filler after the reaction with silane coupling agent (a). In view of enhancing the reactivity with the end capping agent (b), it is therefore preferable to conduct a hydrolysis treatment process for the filler surface-treated with the silane coupling agent (a). The hydrolysis treatment process may involve, for example, allowing for a hydrolysis reaction by diluting the filler with an acetic acid solution (either acetic acid dissolved in water or in a mixed solvent of water and organic solvent) after surface treatment with the silane coupling agent (a).

Another embodiment is, for example, a surface-treated filler for dental use (C-1) resulting from hydrolysis following surface treatment with the silane coupling agent (a) having a polymerizable group.

The filler after the surface treatment with the silane coupling agent (a) (preferably, a filler that has undergone surface treatment with the silane coupling agent (a), and hydrolysis treatment) may be subjected to a surface treatment process with the end capping agent (b).

By conducting a surface treatment for the surface-treated filler for dental use (C-1) with a specific end capping agent (b), it is possible to reduce the unreacted hydrolyzable groups (for example, unreacted hydroxyl groups) after the reaction with the silane coupling agent (a), reducing the reactivity between the filler and polymerizable monomers (particularly, polymerizable monomers with acidic groups) in the dental composition. This enables the resulting dental composition to exhibit excellent storage stability even under severe conditions (for example, 60°C for 4 weeks). The cured product also exhibits sufficient mechanical strength because the polymerizable groups derived from the silane coupling agent (a) and present on the surface of the surface-treated filler for dental use (C-1) provide adequate mechanical strength. By reducing the unreacted hydrolyzable groups (for example, unreacted hydroxyl groups), the present invention can exhibit its effects in compositions containing fillers and polymerizable monomers (particularly, polymerizable monomers with acidic groups), without being restricted to specific dental applications.

Following the surface treatment of the filler, some of the silane coupling agent (a) and end capping agent (b) are physically adsorbed to the filler surface rather than chemically bonded. In view of considerations such as storage stability, it is preferable to include a process of washing away these physically adsorbed components. A method for washing away the physically adsorbed silane coupling agent (a) and end capping agent (b) may involve dissolving the physically adsorbed material in a solvent, and removing the solvent using, for example, a hollow-fiber membrane or filter paper. The washing solvent is not particularly limited. Examples include alcohol-based solvents (such as methanol, ethanol, and isopropanol), acetonitrile, water, and mixed solvents of these.

In the surface treatment process to obtain the surface-treated filler for dental use (C-1), the filler is treated with preferably 0.5 to 40 parts by mass, more preferably 1 to 20 parts by mass, even more preferably 2 to 10 parts by mass of silane coupling agent (a) relative to 100 parts by mass of the filler before surface treatment. When the amount is less than 0.5 parts by mass, it may lead to a failure to sufficiently provide polymerizable groups on the filler surface, potentially resulting in a mechanical strength decrease. When the amount is more than 40 parts by mass, the excessive presence of silane coupling agent (a) may result in a decrease in the mechanical strength of the cured product.

In the surface treatment process to obtain the surface-treated filler for dental use (C-1), the filler is treated with preferably 0.01 to 20 parts by mass, more preferably 0.05 to 15 parts by mass, even more preferably 0.1 to 10 parts by mass of end capping agent (b) relative to 100 parts by mass of the filler before surface treatment. When the amount is less than 0.01 parts by mass, it may lead to a failure to sufficiently treat the filler surface, potentially resulting in the absence of storage stability. When the amount is more than 20 parts by mass, the excessive presence of end capping agent (b) may result in a decrease in the mechanical strength of the cured product.

The surface treatment may accompany polymerization inhibitors to inhibit polymerization of silane coupling agent (a). The polymerization inhibitors may be known polymerization inhibitors, such as 3,5-dibutyl-4-hydroxytoluene (BHT), and p-methoxyphenol (methoquinone).

Preferably, the surface treatment agent used for surface treatment to obtain the surface-treated filler for dental use (C-1) consists solely of silane coupling agent (a) and end capping agent (b). The silane coupling agent (a) and end capping agent (b) may be used with other surface treatment agents, provided that it does not hinder the effectiveness of the present invention. Examples of such other surface treatment agents include organosilazanes such as 1,1,1,3,3,3-hexamethyldisilazane.

The drying process to obtain the surface-treated filler for dental use (C-1) may follow an ordinary method, for example, such as heating, or drying under reduced pressure (vacuum). The heating equipment and vacuum device are not particularly limited, and may be known devices.

A certain preferred embodiment is, for example, a dental composition comprising a polymerizable monomer (A), a polymerization initiator (B), and a filler (C),
wherein the filler (C) comprises a surface-treated filler for dental use (C-1) surface-treated with a silane coupling agent (a) having a polymerizable group, and an end capping agent (b), and
the end capping agent (b) is a compound having a structure represented by the formula (1) or (2) above, and a boiling point of 180°C or less.

A certain embodiment of the present invention is, for example, a surface-treated filler for dental use (C-1) that results from surface treatment of filler with a silane coupling agent (a) having a polymerizable group, and an end capping agent (b), wherein the end capping agent (b) is a compound having a structure represented by the formula (1) or (2) above, and a boiling point of 180°C or less. A certain preferred embodiment is, for example, a surface-treated filler for dental use (C-1) in which the filler comprises an inorganic filler.

In a dental composition of the present invention, the filler (C) may consist solely of a surface-treated filler for dental use (C-1), or may comprise two or more types of surface-treated fillers for dental use (C-1). In a dental composition of the present invention, the content of surface-treated filler for dental use (C-1) is 50 to 2,000 parts by mass relative to 100 parts by mass of the monomer components. The content of surface-treated filler for dental use (C-1) is preferably 100 to 1,000 parts by mass, more preferably 125 to 750 parts by mass, even more preferably 150 parts by mass to 500 parts by mass relative to 100 parts by mass of the monomer components.

In another embodiment, the filler (C) may comprise a surface-treated filler for dental use (C-1), and fillers other than surface-treated filler for dental use (C-1). The fillers other than surface-treated filler for dental use (C-1) may be used alone, or two or more thereof may be used in combination. In such embodiments, the mass ratio of surface-treated filler for dental use (C-1) and fillers other than surface-treated filler for dental use (C-1) is preferably 10:1 to 1:10, more preferably 8:1 to 1:8, even more preferably 1:5 to 5:1. The content of filler (C) is not particularly limited, and the preferred content depends on intended use of the dental composition, as will be described.

A dental composition producing method of the present invention is not particularly limited, as long as it produces a dental composition that comprises a polymerizable monomer (A), a polymerization initiator (B), and a filler (C), and in which the filler (C) comprises a surface-treated filler for dental use (C-1) surface-treated with a silane coupling agent (a) having a polymerizable group, and an end capping agent (b). Such dental compositions can be produced with ease using methods known to a person skilled in the art (for example, such as mixing, and kneading). A dental composition producing method of the present invention is, for example, a method comprising a step of obtaining the surface-treated filler for dental use (C-1), wherein the step of obtaining the surface-treated filler for dental use (C-1) comprises subjecting a filler to surface treatment with the silane coupling agent (a) having a polymerizable group, and subjecting the filler to surface treatment with the end capping agent (b).

### <Polymerization Accelerator (D)>

A dental composition of the present invention may employ a polymerization accelerator (D), along with the polymerization initiator (B). Examples of the polymerization accelerator (D) that may be used in the present invention include amines, sulfinic acid and salts thereof, borate compounds, barbituric acid compounds, triazine compounds, copper compounds, tin compounds, vanadium compounds, halogen compounds, aldehydes, thiol compounds, sulfites, bisulfites, and thiourea compounds.

The amines used as polymerization accelerator (D) can be categorized into aliphatic amines and aromatic amines.

Examples of the aliphatic amines include primary aliphatic amines such as n-butylamine, n-hexylamine, and n-octylamine; secondary aliphatic amines such as diisopropylamine, dibutylamine, and N-methylethanolamine; and tertiary aliphatic amines such as N-methyldiethanolamine, N-ethyldiethanolamine, N-n-butyldiethanolamine, N-lauryldiethanolamine, 2-(dimethylamino)ethyl methacrylate, N-methyldiethanolamine dimethacrylate, N-ethyldiethanolamine dimethacrylate, triethanolamine monomethacrylate, triethanolamine dimethacrylate, triethanolamine trimethacrylate, triethanolamine, trimethylamine, triethylamine, and tributylamine. In view of the curability and storage stability of the dental composition, preferred for use are tertiary aliphatic amines, more preferably N-methyldiethanolamine and triethanolamine.

Examples of the aromatic amines include N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-diisopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, ethyl 4-(N,N-dimethylamino)benzoate, methyl 4-(N,N-dimethylamino)benzoate, propyl 4-(N,N-dimethylamino)benzoate, n-butoxyethyl 4-(N,N-dimethylamino)benzoate, 2-(methacryloyloxy)ethyl 4-(N,N-dimethylamino)benzoate, 4-(N,N-dimethylamino)benzophenone, and butyl 4-(N,N-dimethylamino)benzoate. In view of the ability to impart excellent curability to the dental composition, it is preferable to use at least one selected from the group consisting of N,N-bis(2-hydroxyethyl)-p-toluidine, ethyl 4-(N,N-dimethylamino)benzoate, n-butoxyethyl 4-(N,N-dimethylamino)benzoate, and 4-(N,N-dimethylamino)benzophenone.

Specific examples of the sulfinic acid and salts thereof, borate compounds, barbituric acid compounds, triazine compounds, copper compounds, tin compounds, vanadium compounds, halogen compounds, aldehydes, thiol compounds, sulfites, bisulfites, and thiourea compounds include those mentioned in WO2008/087977.

The polymerization accelerator (D) may be incorporated alone, or two or more thereof may be incorporated in combination.

The content of the polymerization accelerator (D) used in the present invention is not particularly limited. However, in view of considerations such as the curability of the dental composition obtained, the content of polymerization accelerator (D) is preferably 0.001 to 30 parts by mass, more preferably 0.01 to 10 parts by mass, even more preferably 0.05 to 20 parts by mass, particularly preferably 0.1 to 5 parts by mass relative to 100 parts by mass of polymerizable monomer (A) in the dental composition.

When the content of polymerization accelerator (D) is 0.001 parts or more by mass, it allows polymerization to sufficiently proceed, resulting in desired adhesive properties. In this respect, the content of polymerization accelerator (D) is more preferably 0.05 parts or more by mass.

When the content of polymerization accelerator (D) is 30 parts or less by mass, it leads to sufficient adhesive properties. In this respect, the content of polymerization accelerator (D) is more preferably 20 parts or less by mass.

### <Fluorine-Ion Releasing Substance>

A dental composition of the present invention may additionally comprise a fluorine-ion releasing substance. By containing a fluorine-ion releasing substance, the dental composition produced can impart acid resistance to tooth structure. Examples of the fluorine-ion releasing substance include metal fluorides such as sodium fluoride, potassium fluoride, sodium monofluorophosphate, lithium fluoride, and ytterbium fluoride. The fluorine-ion releasing substance may be incorporated alone, or two or more thereof may be incorporated in combination.

A dental composition of the present invention may also comprise known additives, provided that it does not cause a performance drop. Examples of such additives include polymerization inhibitors, antioxidants, pigments, dyes, ultraviolet absorbers, organic solvents, and thickeners. The additives may be used alone, or two or more thereof may be used in combination.

Examples of the polymerization inhibitors include hydroquinone, hydroquinone monomethyl ether, dibutylhydroquinone, dibutylhydroquinone monomethyl ether, t-butylcatechol, 2-t-butyl-4,6-dimethylphenol, 2,6-di-t-butylphenol, and 3,5-di-t-butyl-4-hydroxytoluene. The content of the polymerization inhibitor is preferably 0.001 to 1.0 parts by mass relative to 100 parts by mass of polymerizable monomer (A) in the dental composition.

### <Solvent>

In certain embodiments, a dental composition of the present invention may comprise a solvent. Examples of the solvent include water and organic solvents. Preferably, the solvent is used in the form of a mixed solvent of water and organic solvent. Depending on the embodiment, it may not be required to incorporate organic solvents.

Comprising water in a dental composition of the present invention accelerates the demineralizing effect of polymerizable monomer (A-1) having an acidic group on tooth structure.

It is required to use water that is essentially free of impurities that negatively impact adhesive properties. Preferred for use is distilled water or ion-exchange water. An excessively low water content may result in insufficient promotion of demineralizing effect, whereas an excessively high water content may lead to a decrease in adhesive properties. The content of water among other solvents is preferably 1 to 500 parts by mass, more preferably 5 to 300 parts by mass, even more preferably 10 to 200 parts by mass relative to 100 parts by mass of polymerizable monomer (A) in the dental composition.

When a dental composition of the present invention comprises an organic solvent, it enables greater enhancement of adhesive properties, coatability, and penetration into tooth structure, leading to further prevention of separation of the components in the composition. The organic solvent is typically one having a boiling point of 150°C or less under ordinary pressure, and a solubility in water at 25°C of 5 mass% or more, preferably 30 mass% or more, more preferably a solubility that enables the solvent to dissolve in water in any proportions.

Examples of the organic solvent include methanol, ethanol, 1-propanol, 2-propanol, 1-butanol, 2-methyl-2-propanol, acetone, methyl ethyl ketone, tetrahydrofuran, diethyl ether, diisopropyl ether, hexane, toluene, chloroform, ethyl acetate, and butyl acetate. Considering both biological safety and ease of removal based on volatility, the organic solvent is preferably a water-soluble organic solvent. Specifically, preferred are ethanol, 2-propanol, 2-methyl-2-propanol, acetone, and tetrahydrofuran, more preferably ethanol, 2-propanol, 2-methyl-2-propanol, and tetrahydrofuran.

In certain embodiments, the total solvent content is preferably 1 to 2,000 parts by mass, more preferably 2 to 1,000 parts by mass, even more preferably 3 to 500 parts by mass relative to 100 parts by mass of polymerizable monomer (A) in the dental composition.

In certain embodiments, a dental composition of the present invention preferably comprises each component so as to confine the liquid (composition) pH within a range of 1.5 to 4.0, more preferably 1.8 to 3.5, even more preferably 2.0 to 3.0. When the pH of the composition is below 1.5, there is a possibility of excessive demineralization during total etching, a process applied to tooth surface following phosphoric acid etching treatment, potentially resulting in a decrease in adhesion. Conversely, when the pH of the composition is above 4.0, there is a possibility of decreased adhesion during self etching due to reduced demineralizing action.

A dental composition of the present invention can be used for dental treatments involving, for example, dental cements, dental bonding materials (for example, orthodontic bonding materials), dental composite resins (for example, self-adhesive dental composite resins), pit and fissure sealants, loose tooth fixing materials, and dental abutment construction materials. The preferred applications are self-adhesive dental composite resins, dental cements, dental composite resins (excluding self-adhesive dental composite resins), dental abutment construction materials, and dental bonding materials. In its use in these applications, a dental composition of the present invention, by comprising the surface-treated filler for dental use (C-1), can also exhibit excellent acid resistance, and prevent reaction with polymerizable monomers (particularly, polymerizable monomers with acidic groups). Moreover, because there is no situation where the other components impede the effects of including the surface-treated filler for dental use (C-1), a dental composition of the present invention shows excellent storage stability even under severe conditions (for example, 60°C for 4 weeks). With the surface-treated filler for dental use (C-1), the cured product also exhibits sufficient mechanical strength in these applications because the polymerizable groups derived from the silane coupling agent (a) and present on the surface provide adequate mechanical strength. For these applications, a dental composition of the present invention may be provided as a single-bottle or single-paste composition by merging its components, or a two-bottle or two-paste composition by dividing the components into two parts. The following describes specific embodiments of the dental composition in different applications.

### <Self-Adhesive Dental Composite Resin>

A preferred embodiment of a dental composition of the present invention is, for example, a self-adhesive dental composite resin.

When used as a self-adhesive dental composite resin, it is preferable that a dental composition of the present invention comprise a polymerizable monomer (A), a polymerization initiator (B), a surface-treated filler for dental use (C-1), and a polymerization accelerator (D), and the polymerizable monomer (A) comprise a polymerizable monomer (A-1) having an acidic group, a hydrophobic polymerizable monomer (A-2a) having no acidic group, and an hydrophilic polymerizable monomer (A-2b) having no acidic group. Preferably, the polymerization initiator (B) comprises a photopolymerization initiator. More preferably, the polymerization initiator (B) comprises a water-soluble photopolymerization initiator (B-1) and a water-insoluble photopolymerization initiator (B-2). When used as a self-adhesive dental composite resin, a dental composition of the present invention may employ a pretreatment material. However, use of pretreatment materials is not necessary because the self-adhesive properties of the composition eliminate the necessity for pretreatment materials. The self-adhesive dental composite resin may consist solely of a dental composition of the present invention, without pretreatment materials.

Concerning the content of each component in the self-adhesive dental composite resin, it is preferable to comprise 1 to 50 parts by mass of polymerizable monomer (A-1) having an acidic group, 20 to 99 parts by mass of hydrophobic polymerizable monomer (A-2a) having no acidic group, and 0 to 50 parts by mass of hydrophilic polymerizable monomer (A-2b) having no acidic group, more preferably 1 to 40 parts by mass of polymerizable monomer (A-1) having an acidic group, 40 to 99 parts by mass of hydrophobic polymerizable monomer (A-2a) having no acidic group, and 0 to 40 parts by mass of hydrophilic polymerizable monomer (A-2b) having no acidic group, even more preferably 1 to 30 parts by mass of polymerizable monomer (A-1) having an acidic group, 60 to 99 parts by mass of hydrophobic polymerizable monomer (A-2a) having no acidic group, and 0 to 30 parts by mass of hydrophilic polymerizable monomer (A-2b) having no acidic group in 100 parts by mass of polymerizable monomer (A) in the dental composition. It is also preferable to comprise 0.001 to 30 parts by mass of polymerization initiator (B), 50 to 2,000 parts by mass of surface-treated filler for dental use (C-1), and 0.001 to 20 parts by mass of polymerization accelerator (D), more preferably 0.05 to 10 parts by mass of polymerization initiator (B), 100 to 1,000 parts by mass of surface-treated filler for dental use (C-1), and 0.05 to 10 parts by mass of polymerization accelerator (D) relative to 100 parts by mass of polymerizable monomer (A). The dental composition used as a self-adhesive dental composite resin is not required to comprise a hydrophilic polymerizable monomer (A-2b).

### <Dental Cement>

Another preferred embodiment of a dental composition of the present invention is, for example, a dental cement. Preferred examples of the dental cement include resin cements, glass ionomer cements, and resin-reinforced glass ionomer cements.

When using the dental cement, pretreatment materials such as self-etching primers may be used in advance. When used as a dental cement, it is preferable that a dental composition of the present invention comprise a polymerizable monomer (A), a polymerization initiator (B), a surface-treated filler for dental use (C-1), and a polymerization accelerator (D), and the polymerizable monomer (A) comprise a polymerizable monomer (A-1) having an acidic group, a hydrophobic polymerizable monomer (A-2a) having no acidic group, and a hydrophilic polymerizable monomer (A-2b) having no acidic group. Preferably, the polymerization initiator (B) comprises a chemical polymerization initiator. More preferably, the polymerization initiator (B) is a combination of a chemical polymerization initiator and a photopolymerization initiator. Preferably, the photopolymerization initiator is a combination of water-soluble photopolymerization initiator (B-1) and water-insoluble photopolymerization initiator (B-2).

Concerning the content of each component in the dental cement, it is preferable to comprise 0 to 50 parts by mass of polymerizable monomer (A-1) having an acidic group, 50 to 99 parts by mass of hydrophobic polymerizable monomer (A-2a) having no acidic group, and 0 to 50 parts by mass of hydrophilic polymerizable monomer (A-2b) having no acidic group, more preferably 0 to 40 parts by mass of polymerizable monomer (A-1) having an acidic group, 60 to 99 parts by mass of hydrophobic polymerizable monomer (A-2a) having no acidic group, and 0 to 40 parts by mass of hydrophilic polymerizable monomer (A-2b) having no acidic group, even more preferably 0 to 30 parts by mass of polymerizable monomer (A-1) having an acidic group, 70 to 99 parts by mass of hydrophobic polymerizable monomer (A-2a) having no acidic group, and 0 to 30 parts by mass of hydrophilic polymerizable monomer (A-2b) having no acidic group in 100 parts by mass of polymerizable monomer (A) in the dental composition. It is also preferable to comprise 0.001 to 30 parts by mass of polymerization initiator (B), 50 to 2,000 parts by mass of surface-treated filler for dental use (C-1), and 0.001 to 20 parts by mass of polymerization accelerator (D), more preferably 0.05 to 10 parts by mass of polymerization initiator (B), 100 to 1,500 parts by mass of surface-treated filler for dental use (C-1), and 0.05 to 10 parts by mass of polymerization accelerator (D) relative to 100 parts by mass of polymerizable monomer (A). The dental cement is not required to comprise a hydrophilic polymerizable monomer (A-2b). When used as a type of dental cement used with a pretreatment material, it is not required to comprise a polymerizable monomer (A-1) having an acidic group.

### <Dental Bonding Material>

A preferred embodiment of a dental composition of the present invention is, for example, a dental bonding material. The dental bonding material allows demineralization, penetration, and cure to be performed in a single step.

The dental bonding material may be a two-bottle type, which is used by mixing separately prepared two components, A and B, immediately before use, or a one-bottle type, where the components can be directly used from one bottle. The one-bottle type involves a simpler process, and has more merits in use. The dental bonding material may use, for example, a self-etching primer as a pretreatment material. When used as such dental bonding materials, it is preferable that the dental composition comprise a polymerizable monomer (A), a polymerization initiator (B), a surface-treated filler for dental use (C-1), and a polymerization accelerator (D), and the polymerizable monomer (A) comprise a polymerizable monomer (A-1) having an acidic group, a hydrophobic polymerizable monomer (A-2a) having no acidic group, a hydrophilic polymerizable monomer (A-2b) having no acidic group, and a solvent.

Preferably, the polymerization initiator (B) comprises a photopolymerization initiator. More preferably, the polymerization initiator (B) comprises a water-soluble photopolymerization initiator (B-1) and a water-insoluble photopolymerization initiator (B-2).

Concerning the content of each component in the dental bonding material, it is preferable to comprise 1 to 90 parts by mass of polymerizable monomer (A-1) having an acidic group, 40 to 96 parts by mass of hydrophobic polymerizable monomer (A-2a) having no acidic group, and 3 to 50 parts by mass of hydrophilic polymerizable monomer (A-2b) having no acidic group, more preferably 1 to 40 parts by mass of polymerizable monomer (A-1) having an acidic group, 50 to 90 parts by mass of hydrophobic polymerizable monomer (A-2a) having no acidic group, and 5 to 40 parts by mass of hydrophilic polymerizable monomer (A-2b) having no acidic group, even more preferably 5 to 30 parts by mass of polymerizable monomer (A-1) having an acidic group, 60 to 80 parts by mass of hydrophobic polymerizable monomer (A-2a) having no acidic group, and 15 to 35 parts by mass of hydrophilic polymerizable monomer (A-2b) having no acidic group in 100 parts by mass of polymerizable monomer (A) in the dental composition. It is also preferable to comprise 0.001 to 30 parts by mass of water-soluble photopolymerization initiator (B-1), 0.001 to 30 parts by mass of water-insoluble photopolymerization initiator (B-2), 0.001 to 20 parts by mass of polymerization accelerator (D), 1 to 100 parts by mass of surface-treated filler for dental use (C-1), and 1 to 2,000 parts by mass of solvent, more preferably 0.05 to 10 parts by mass of water-soluble photopolymerization initiator (B-1), 0.05 to 10 parts by mass of water-insoluble photopolymerization initiator (B-2), 0.05 to 10 parts by mass of polymerization accelerator (D), 3 to 75 parts by mass of surface-treated filler for dental use (C-1), and 2 to 1,000 parts by mass of solvent relative to 100 parts by mass of polymerizable monomer (A).

### <Dental Composite Resin>

A preferred embodiment of a dental composition of the present invention is, for example, a dental composite resin (excluding self-adhesive dental composite resins). When used as a dental composite resin, a dental composition of the present invention comprises a polymerizable monomer (A), a polymerization initiator (B), a surface-treated filler for dental use (C-1), and a polymerization accelerator (D). Preferably, the polymerization initiator (B) comprises a photopolymerization initiator. More preferably, the polymerization initiator (B) comprises a water-soluble photopolymerization initiator (B-1) and a water-insoluble photopolymerization initiator (B-2).

Concerning the content of each component in the dental composite resin, it is preferable to comprise 20 to 100 parts by mass of hydrophobic polymerizable monomer (A-2a) having no acidic group, and 0 to 80 parts by mass of hydrophilic polymerizable monomer (A-2b) having no acidic group, more preferably 40 to 100 parts by mass of hydrophobic polymerizable monomer (A-2a) having no acidic group, and 0 to 40 parts by mass of hydrophilic polymerizable monomer (A-2b) having no acidic group, even more preferably 60 to 100 parts by mass of hydrophobic polymerizable monomer (A-2a) having no acidic group, and 0 to 30 parts by mass of hydrophilic polymerizable monomer (A-2b) having no acidic group in 100 parts by mass of polymerizable monomer (A) in the dental composition. It is also preferable to comprise 0.001 to 30 parts by mass of polymerization initiator (B), 50 to 2,000 parts by mass of surface-treated filler for dental use (C-1), and 0.001 to 20 parts by mass of polymerization accelerator (D), more preferably 0.05 to 10 parts by mass of polymerization initiator (B), 100 to 1,500 parts by mass of surface-treated filler for dental use (C-1), and 0.05 to 10 parts by mass of polymerization accelerator (D) relative to 100 parts by mass of polymerizable monomer (A). The dental composition used as a dental composite resin is not required to comprise a hydrophilic polymerizable monomer (A-2b).

### <Dental Abutment Construction Material>

Another preferred embodiment of a dental composition of the present invention is, for example, a dental abutment construction material. Preferred examples of the dental abutment construction material include resin cements. The dental abutment construction material may use pretreatment materials such as self-etching primers. When used as a dental abutment construction material, it is preferable that a dental composition of the present invention comprise a polymerizable monomer (A), a polymerization initiator (B), a surface-treated filler for dental use (C-1), and a polymerization accelerator (D), and the polymerizable monomer (A) comprise a polymerizable monomer (A-1) having an acidic group, a hydrophobic polymerizable monomer (A-2a) having no acidic group, and a hydrophilic polymerizable monomer (A-2b) having no acidic group. Preferably, the polymerization initiator (B) comprises a chemical polymerization initiator. More preferably, the polymerization initiator (B) is a combination of a chemical polymerization initiator and a photopolymerization initiator. Preferably, the photopolymerization initiator is a combination of water-soluble photopolymerization initiator (B-1) and water-insoluble photopolymerization initiator (B-2).

Concerning the content of each component in the dental abutment construction material, it is preferable to comprise 0 to 50 parts by mass of polymerizable monomer (A-1) having an acidic group, 50 to 99 parts by mass of hydrophobic polymerizable monomer (A-2a) having no acidic group, and 0 to 50 parts by mass of hydrophilic polymerizable monomer (A-2b) having no acidic group, more preferably 0 to 40 parts by mass of polymerizable monomer (A-1) having an acidic group, 60 to 99 parts by mass of hydrophobic polymerizable monomer (A-2a) having no acidic group, and 0 to 40 parts by mass of hydrophilic polymerizable monomer (A-2b) having no acidic group, even more preferably 0 to 30 parts by mass of polymerizable monomer (A-1) having an acidic group, 70 to 99 parts by mass of hydrophobic polymerizable monomer (A-2a) having no acidic group, and 0 to 30 parts by mass of hydrophilic polymerizable monomer (A-2b) having no acidic group in 100 parts by mass of polymerizable monomer (A) in the dental composition. It is also preferable to comprise 0.001 to 30 parts by mass of polymerization initiator (B), 50 to 2,000 parts by mass of surface-treated filler for dental use (C-1), and 0.001 to 20 parts by mass of polymerization accelerator (D), more preferably 0.05 to 10 parts by mass of polymerization initiator (B), 100 to 1,500 parts by mass of surface-treated filler for dental use (C-1), and 0.05 to 10 parts by mass of polymerization accelerator (D) relative to 100 parts by mass of polymerizable monomer (A). The dental abutment construction material is not required to comprise a hydrophilic polymerizable monomer (A-2b). When used as a type of dental abutment construction material used with a pretreatment material, it is not required to comprise a polymerizable monomer (A-1) having an acidic group.

In any of the preferred embodiments as self-adhesive dental composite resins, dental cements, dental composite resins, dental abutment construction materials, and dental bonding materials, the content of each component may be varied as appropriate based on the descriptions provided in this specification, and changes such as addition and deletion can be made for any of the components.

The present invention encompasses embodiments combining the foregoing features in various ways within the technical idea of the present invention, provided that the present invention can exhibit its effects.

### EXAMPLES

The following describes the present invention in greater detail through Examples and Comparative Examples. However, the present invention is not limited by the following Examples. In the following, "part(s)" means part(s) by mass, unless otherwise specifically stated.

The components of the dental compositions of Examples and Comparative Examples are presented below, along with the abbreviations.

### [Polymerizable monomer (A-1) having an acidic group]

MDP: 10-methacryloyloxydecyl dihydrogen phosphate

### [Polymerizable monomer (A-2) having no acidic group]

D-2.6E: 2,2-bis(4-methacryloyloxypolyethoxyphenyl)propane (with an average of 2.6 moles of ethoxy group added)
3G: triethylene glycol dimethacrylate
MAEA: N-methacryloyloxyethylacrylamide

### [Polymerization initiator (B)]

### · Water-soluble photopolymerization initiator (B-1)

Li-TPO: Lithium phenyl(2,4,6-trimethylbenzoyl)phosphinate

### · Water-insoluble photopolymerization initiator (B-2)

CQ: camphorquinone

### [Surface-treated filler for dental use (C-1)]

Production Example 1: Production of Filler 1

### · Hydrochloric acid treatment process

One-hundred grams of 8235 UF0.7 grade (barium glass manufactured by SCHOTT; average particle diameter: 0.7 µm) was added into a beaker with 500 mL of a 5 mass% hydrochloric acid aqueous solution. The mixture was stirred at room temperature for 30 minutes, and filtered under reduced pressure to remove the hydrochloric acid aqueous solution. Subsequently, distilled water (500 mL) and filler (acid-treated material) were added into the beaker, stirred at room temperature for 30 minutes, and filtered under reduced pressure to remove the aqueous solution. This washing process was repeated three times, followed by vacuum drying.

### · Surface treatment process with silane coupling agent (a)

A three-neck flask was charged with 100 g of the filler treated with hydrochloric acid, 10 g of 3-methacryloyloxypropyltrimethoxysilane, and 200 mL of a 0.3 mass% acetic acid aqueous solution, and these were stirred at room temperature for 2 hours. After removing water by freeze drying, a heat treatment was carried out at 80°C for 5 hours. Subsequently, 500 mL of ethanol and 100 g of the filler (surface-treated with silane coupling agent (a)) were added into a beaker, stirred at room temperature for 30 minutes, and filtered under reduced pressure to remove the solution. This washing process was repeated three times, followed by vacuum drying.

### · Hydrolysis treatment process

One-hundred grams of the filler (surface-treated with silane coupling agent (a)) was added into a beaker with a 5 mass% acetic acid solution (5 g of acetic acid, 100 mL of a mixed solution of water and ethanol (50:50 (v/v))). The mixture was stirred at room temperature for 30 minutes, and filtered under reduced pressure to remove the solvent. Subsequently, 500 mL of a separately prepared water/ethanol mixed solution (50:50 (v/v)) and the filler (after hydrolysis treatment) were added into a beaker, stirred at room temperature for 30 minutes, and filtered under reduced pressure to remove the solvent. This washing process was repeated three times, followed by vacuum drying.

### · Surface treatment process with end capping agent (b)

One-hundred grams of the filler (after hydrolysis treatment) was added into a pressure tight vessel with 1 g of hexamethylcyclotrisiloxane. These were allowed to react at 140°C for 24 hours. Subsequently, 500 mL of ethanol and 100 g of the filler (surface-treated with hexamethylcyclotrisiloxane (end capping agent (b)) were added into a beaker, stirred at room temperature for 30 minutes, and filtered under reduced pressure to remove the solvent. This washing process was repeated three times, followed by vacuum drying to yield filler 1.

### Production Example 2: Production of Filler 2

Filler 2 was produced using the same method employed in Production Example 1, except that the raw material 8235 UF0.7 grade was replaced with GM27884 NF180 grade (barium glass manufactured by SCHOTT; average particle diameter: 0.18 µm).

### Production Example 3: Production of Filler 3

Barium glass (product code E-3000 manufactured by ESSTECH) was pulverized with a ball mill to obtain a barium glass powder. The barium glass powder had an average particle diameter of 2.4 µm as measured by volume with a laser diffraction particle size distribution analyzer (Model SALD-2300 manufactured by Shimadzu Corporation). Filler 3 was produced using the same method employed in Production Example 1, except that this filler was used as a raw material, instead of 8235 UF0.7 grade.

### Production Example 4: Production of Filler 4

A silica stone powder (a quartz manufactured by Nitchitsu Co., Ltd. under the trade name Hi-Silica) was pulverized with a ball mill to obtain a pulverized silica stone powder. The pulverized silica stone powder had an average particle diameter of 2.2 µm as measured by volume with a laser diffraction particle size distribution analyzer (Model SALD-2300 manufactured by Shimadzu Corporation). Filler 4 was produced using the same method employed in Production Example 1, except that this filler was used as a raw material, instead of 8235 UF0.7 grade.

### Production Example 5: Production of Filler 5

Filler 5 was produced using the same raw materials and method employed in Production Example 1, except that the hydrolysis treatment process was omitted.

### Production Example 6: Production of Filler 6

Filler 6 was produced using the same raw materials and method employed in Production Example 1, except that the hydrochloric acid treatment process was omitted.

### Production Example 7: Production of Filler 7

Filler 7 was produced through the hydrochloric acid treatment process using 8235 UF0.7 grade. In the subsequent surface treatment process following Production Example 1, the filler was treated with the end capping agent (b) first, followed by the silane coupling agent (a).

### Production Example 8: Production of Filler 8

Filler 8 was produced through the hydrolysis treatment process and the surface treatment process with end capping agent (b) following the method of Production Example 1, using a surface-treated silica-zirconia oxide aggregate filler (manufactured by Sukgyung AT under the trade name SG-SZ200G151CMP8; average particle diameter of primary particles: 200 nm, average particle diameter of secondary particles: 5.2 µm, refractive index: 1.51, constituent components: SiO₂, ZrO₂).

### [Filler (C) other than surface-treated filler for dental use (C-1)]

### Production Example 9: Production of Filler 9

Filler 9 was produced using the same raw materials and method employed in Production Example 1, except that the surface treatment process with silane coupling agent (a), and the hydrolysis treatment process were omitted.

### Production Example 10: Production of Filler 10

Filler 10 was produced by treating 8235 UF0.7 grade only through the surface treatment process with silane coupling agent (a), following Production Example 1.

### Production Example 11: Production of Filler 11

Filler 11 was produced by treating 8235 UF0.7 grade only through the hydrochloric acid treatment process, and the surface treatment process with silane coupling agent (a), following Production Example 1.

### Production Example 12: Production of Filler 12

Filler 12 was produced by treating GM27884 NF180 grade only through the hydrochloric acid treatment process, and the surface treatment process with silane coupling agent (a), following Production Example 1.

### Production Example 13: Production of Filler 13

Filler 13 was produced through the process described below, after treating 8235 UF0.7 grade through the hydrochloric acid treatment process, the surface treatment process with silane coupling agent (a), and the hydrolysis treatment process following Production Example 1.

### · Surface treatment process with dimethyldimethoxysilane

A three-neck flask was charged with 100 g of the filler obtained through the foregoing process, along with 5 g of dimethyldimethoxysilane, and 200 mL of a 0.3 mass% acetic acid ethanol solution. These were stirred at room temperature for 2 hours. After removing the solvent under reduced pressure by distillation, the material was dried in a vacuum, and subjected to a heat treatment at 80°C for 5 hours. Subsequently, 500 mL of ethanol and 100 g of the filler (surface-treated with dimethyldimethoxysilane) were added into a beaker, stirred at room temperature for 30 minutes, and filtered under reduced pressure to remove the solution. This washing process was repeated three times, followed by vacuum drying.

### Production Example 14: Production of Filler 14

Filler 14 was produced through the process described below, after treating 8235 UF0.7 grade through the hydrochloric acid treatment process, the surface treatment process with silane coupling agent (a), and the hydrolysis treatment process following Production Example 1.

### · Surface treatment process with 1,1,1,3,3,3-hexamethyldisilazane

One-hundred grams of the filler obtained through the foregoing process was added into a three-neck flask with 200 mL of isopropanol, and 4 g of 1,1,1,3,3,3-hexamethyldisilazane. These were allowed to react at 40°C for 72 hours. Subsequently, 5 g of a 35% hydrochloric acid aqueous solution was added to the total amount of the mixture resulting from the surface treatment, allowing the filler (surface-treated with 1,1,1,3,3,3-hexamethyldisilazane) to precipitate. After the filtration of the precipitate with filter paper, 500 mL of distilled water and the precipitate were added into a beaker, stirred at room temperature for 30 minutes, and filtered under reduced pressure to remove the aqueous solution. This washing process was repeated three times, followed by vacuum drying at 100°C to yield filler 14.

### Production Example 15: Production of Filler 15

One-hundred grams of OX50 (ultrafine particulate silica Aerosil^{®} OX50 manufactured by Nippon Aerosil Co., Ltd.; average particle diameter: 0.04 µm) was added into a three-neck flask, along with 7 g of 3-methacryloyloxypropyltrimethoxysilane, and 200 mL of a 0.3 mass% acetic acid aqueous solution. These were stirred at room temperature for 2 hours. After removing water by freeze drying, a heat treatment was carried out at 80°C for 5 hours to yield filler 15 (silane-treated silica).

### [Polymerization accelerator (D)]

DABE: ethyl 4-(N,N-dimethylamino)benzoate

### [Polymerization inhibitor]

BHT: 3,5-di-t-butyl-4-hydroxytoluene

### Examples 1-1 to 1-11 and Comparative Examples 1-1 to 1-6 (Preparation of Dental Composition)

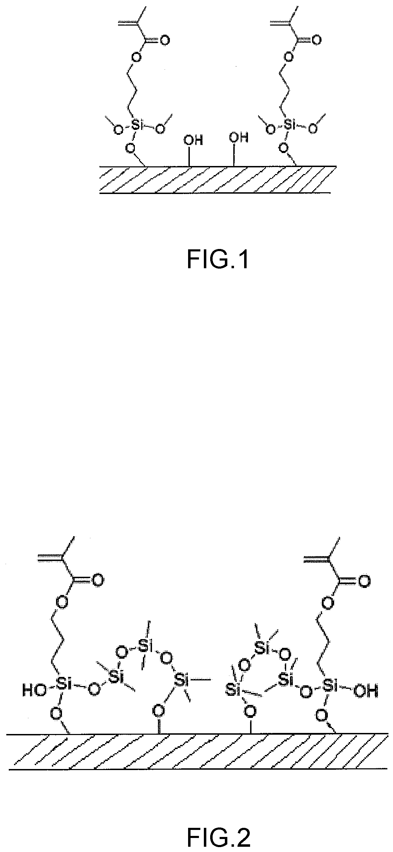

The raw materials presented in Tables 1 and 2 were mixed and kneaded at ordinary temperature (23°C) in the dark to prepare paste-form dental compositions (dental composite resins). The properties of these compositions were then examined following the methods of Test Examples 1 and 2 below. The properties of the fillers produced following Production Examples were also examined according to the method of Test Example 3. The results are presented in Tables 1 and 2. FIG. 1 shows the surface structure of the surface-treated filler (filler 9) obtained in Production Example 9. FIG. 2 shows the surface structure of the surface-treated filler for dental use (C-1) (filler 1) obtained in Production Example 1.

### Test Example 1: Flexural Strength

The flexural strength was evaluated by a flexure test in compliance with ISO 4049: 2009, specifically as follows. The prepared paste (dental composition) was filled into a SUS die (2 mm in length × 25 mm in width × 2 mm in thickness), and the top and bottom surfaces (2 mm × 25 mm) of the paste were pressed with glass slides. Subsequently, the paste was cured by applying light to the top and bottom of the paste through the glass slides at five points on each side, 10 seconds per point, using a dental visible-light irradiator PenCure 2000 (manufactured by J. Morita Corp.). The resulting cured product was measured for three-point flexural strength by conducting a flexure test at a span length of 20 mm and a crosshead speed of 1 mm/min, using a universal testing machine (Autograph AG-I 100kN, manufactured by Shimadzu Corporation), and the average value was calculated (n = 5).

### Test Example 2: Shear Adhesion Test on Bovine Dentin

The labial surface of a bovine mandibular incisor was ground with #80 silicon carbide paper (manufactured by Nihon Kenshi Co., Ltd.) under running water to obtain a sample with an exposed flat dentin surface. The sample tooth prepared in this fashion was secured to the tape attached to the bottom of a mold having 15 holes (a 15-hole mold, manufactured by Ultradent Products Inc.; 35 mm in diameter × 25 mm in height). After filling a plaster into the mold, the plaster was left to stand for about 30 minutes to harden, producing a composite of bovine mandibular incisor and plaster. The composite was then taken out of the mold as a sample.

The sample had the bovine mandibular incisor exposed at the top of the plaster. The exposed surface of bovine mandibular incisor was polished with #600 silicon carbide paper(manufactured by Nihon Kenshi Co., Ltd.) under running water until the polished surface was large enough to provide a bonding surface (Ø = 2.38 mm or more). The bonding surface was then ultrasonically washed with water for 5 minutes.

Subsequently, a CR filling mold (Bonding Mold Insert, manufactured by Ultradent Products Inc.; Ø = 2.38 mm) was installed on a dedicated instrument (Bonding Clamp, manufactured by Ultradent Products Inc.). The mold was then lowered to allow it to contact the bonding surface of the sample. Thereafter, the dental composition, immediately after production in each Example and Comparative Example, was filled into the hole of the mold to form a thin layer of no greater than 1 mm. This was immediately followed by filling another portion of the dental composition into the mold (to about 2/3 of the mold, or about 2 mm thick). The dental composition was left to stand for 10 seconds, and irradiated with light for 10 seconds to cure, using a dental LED photoirradiator (manufactured by Ultradent Products Inc. under the trade name VALO). The sample was removed from the mold, and used as an adhesion test sample. The adhesion test sample was immersed in distilled water inside a sample container, and was left to stand for 24 hours in this state in a thermostatic chamber that had been set to 37°C. The sample was measured for its bond strength after being taken out of the chamber.

For the measurement of bond strength (shear bond strength), the adhesion test sample was installed on a dedicated holder (Test Base Clamp, manufactured by Ultradent Products Inc.), and the bond strength was measured using a dedicated jig (Crosshead Assembly, manufactured by Ultradent Products Inc.) and a universal testing machine (manufactured by Shimadzu Corporation) with the crosshead speed set at 1 mm/min. The average value was calculated for n = 10.

Separately, the same shear adhesion test was conducted on bovine dentin, except that the dental compositions prepared in Examples and Comparative Examples were tested after being stored at 60°C for 4 weeks, instead of immediately after production.

In Tables 1 and 2, "Initial samples" refers to paste-form dental compositions measured for shear bond strength after being prepared into adhesion test samples immediately after production, and ʺʺ60°C 4-week samples" refers to paste-form dental compositions measured for shear bond strength after being prepared into adhesion test samples following 4 weeks of storage at 60°C.

### Test Example 3: Acid Resistance Test

For each of fillers 1 to 14, 0.5 g of filler was added to a 5% acetic acid aqueous solution. This mixture underwent ultrasonic dispersion for 5 minutes using a Branson Tabletop ultrasonic cleaner Bransonic^{®} M2800-J, 110 W, manufactured by Yamato Scientific Co., Ltd.), and was left to stand at room temperature for 6 hours. To remove the filler, the mixture was centrifuged at 20,000 rpm for 30 minutes using a Himac CR21GII, manufactured by Eppendorf-Himac Technologies Co., Ltd., allowing the filler to settle. The supernatant was immediately collected without delay. After passing the supernatant through a membrane filter (with a pore size of 0.45 µm) to remove impurities, an elemental analysis was conducted using an ICP emission spectrometer (Thermo Fisher Scientific iCAP6500 Duo manufactured by Thermo Fisher Scientific Inc.; RF power: 1,150 W, auxiliary gas flow rate: 0.5 L/min, nebulizer gas flow rate: 0.7 L/min, pump flow rate: 50 rpm, plasma view: axial, low wavelength range: 15 sec, high wavelength range: 10 sec) (n = 3). The average values were calculated from the measured quantities of metallic elements (unit: ppm by mass). The results are presented in Tables 1 and 2.

The acid resistance evaluation did not include filler 15, and the acid resistance tests for fillers (C) other than surface-treated filler for dental use (C-1) in Table 2 reflect the results of acid resistance tests conducted for fillers 9 to 14. Because a lower amount of detected metallic elements in the acid resistance test is suggestive of reduced interaction between the acidic component and the filler, this indicates that the filler surface is more densely coated with organic molecules.

**[Table 1]**

| Components (parts by mass) | | Ex. 1-1 | Ex. 1-2 | Ex. 1-3 | Ex. 14 | Ex. 1-5 | Ex. 1-6 | Ex. 1-7 | Ex. 1-8 | Ex. 1-9 | Ex. 1-10 | Ex. 1-11 |
|---|---|---|---|---|---|---|---|---|---|---|---|---|
| Polymerizable monomer (A-1) having acidic group | MDP | 5 | 5 | 5 | 5 | 5 | 5 | 5 | 5 | 15 | 35 | 5 |
| Polymerizable monomer (A-2) having no acidic group | D-2.6E | 60 | 60 | 60 | 60 | 60 | 60 | 60 | 60 | 60 | 50 | 60 |
| | 3G | 30 | 30 | 30 | 30 | 30 | 30 | 30 | 30 | 20 | 10 | 30 |
| | MAEA | 5 | 5 | 5 | 5 | 5 | 5 | 5 | 5 | 5 | 5 | 5 |
| Polymerization initiator (B) | CQ | 0.3 | 0.3 | 0.3 | 0.3 | 0.3 | 0.3 | 0.3 | 0.3 | 0.3 | 0.3 | 0.3 |
| | Li-TPO | - | - | - | - | 0.5 | - | - | - | - | - | - |
| Surface-treated filler for dental use (C-1) | Filler 1 | 300 | - | - | - | - | - | - | - | 300 | 300 | - |
| | Filler 2 | - | 260 | - | - | - | - | - | - | - | - | - |
| | Filler 3 | - | - | 320 | - | - | - | - | - | - | - | - |
| | Filler 4 | - | - | - | 320 | 320 | - | - | - | - | - | - |
| | Filler 5 | - | - | - | - | - | 300 | - | - | - | - | - |
| | Filler 6 | - | - | - | - | - | - | 300 | - | - | - | - |
| | Filler 7 | - | - | - | - | - | - | - | 300 | - | - | - |
| | Filler 8 | - | - | - | - | - | - | - | - | - | - | 300 |
| Filler other than surface-treated filler for dental use (C-1) | Filler 15 | 15 | 15 | 15 | 15 | 15 | 15 | 15 | 15 | 15 | 15 | 15 |
| Polymerization accelerator (D) | DABE | 0.4 | 0.4 | 0.4 | 0.4 | 0.4 | 0.4 | 0.4 | 0.4 | 0.4 | 0.4 | 0.4 |
| Polymerization inhibitor | BHT | 0.05 | 0.05 | 0.05 | 0.05 | 0.05 | 0.05 | 0.05 | 0.05 | 0.05 | 0.05 | 0.05 |
| Flexural strength (MPa) | | 130 | 118 | 132 | 146 | 150 | 103 | 126 | 94 | 113 | 105 | 120 |
| Shear adhesion test on bovine dentin (MPa) | Initial samples | 7 | 6 | 7 | 8 | 12 | 6 | 6 | 5 | 9 | 8 | 7 |
| | 60°C 4-week samples | 6 | 5 | 7 | 6 | 10 | 5 | 5 | 5 | 9 | 6 | 6 |
| Acid resistance test for surface-treated filler for dental use (C-1) (ppm) | | 23 | 44 | 18 | 6 | 6 | 70 | 93 | 10 | 23 | 23 | 8 |

**[Table 2]**

| Components (parts by mass) | | Com. Ex. 1-1 | Com. Ex. 1-2 | Com. Ex. 1-3 | Com. Ex. 1-4 | Com. Ex. 1-5 | Com. Ex. 1-6 |
|---|---|---|---|---|---|---|---|
| Polymerizable monomer (A-1) having acidic group | MDP | 5 | 5 | 5 | 5 | 5 | 5 |
| Polymerizable monomer (A-2) having no acidic group | D-2.6E | 60 | 60 | 60 | 60 | 60 | 60 |
| | 3G | 30 | 30 | 30 | 30 | 30 | 30 |
| | MAEA | 5 | 5 | 5 | 5 | 5 | 5 |
| Polymerization initiator (B) | CQ | 0.3 | 0.3 | 0.3 | 0.3 | 0.3 | 0.3 |
| Surface-treated filler for dental use (C-1) | Filler 1 | - | - | - | - | - | - |
| Filler (C) other than surface-treated filler for dental use (C-1) | Filler 9 | 300 | - | - | - | - | - |
| | Filler 10 | - | 300 | - | - | - | - |
| | Filler 11 | - | - | 260 | - | - | - |
| | Filler 12 | - | - | - | 300 | - | - |
| | Filler 13 | - | - | - | - | 300 | - |
| | Filler 14 | - | - | - | - | - | 300 |
| | Filler 15 | 15 | 15 | 15 | 15 | 15 | 15 |
| Polymerization accelerator (D) | DABE | 0.4 | 0.4 | 0.4 | 0.4 | 0.4 | 0.4 |
| Polymerization inhibitor | BHT | 0.05 | 0.05 | 0.05 | 0.05 | 0.05 | 0.05 |
| Flexural strength (MPa) | | 60 | 128 | 124 | 120 | 123 | 126 |
| Shear adhesion test on bovine dentin (MPa) | Initial samples | 4 | 6 | 5 | 6 | 6 | 6 |
| | 60°C 4-week samples | 4 | 0 | 0 | 1 | 0 | 0 |
| Acid resistance test for filler (C) other than surface-treated filler for dental use (C-1) (ppm) | | 13 | 870 | 1200 | 560 | 340 | 300 |

As can be seen from the results presented in Table 1, the dental compositions of Examples exhibited superior mechanical strength with a flexural strength of 94 MPa or more in their cured products. The dental compositions of Examples also showed outstanding storage stability with a shear bond strength of 5 MPa or more to bovine dentin, even after 4 weeks of storage at 60°C.

This is in contrast to the dental compositions of Comparative Examples 1-1 to 1-6 with no surface-treated filler for dental use (C-1). As shown in Table 2, the dental composition of Comparative Example 1-1 had an insufficient flexural strength of 60 MPa. In Comparative Examples 1-2 to 1-6, the bond strength to bovine dentin was 1 MPa or less after 4 weeks of storage at 60°C, confirming low storage stability under severe conditions.

The filler 11 used in Comparative Example 1-3 and the filler 12 used in Comparative Example 1-4, treated with hydrochloric acid and involving surface treatment with silane coupling agent (a), correspond to Patent Literature 3 (JP 2011-178778 A).

The filler 14 used in Comparative Example 1-6 corresponds to Patent Literature 4 (WO2018/074594). In the acid resistance test, Comparative Examples 1-2 to 1-6 with fillers other than surface-treated filler for dental use (C-1) showed a high dissolution rate of 300 ppm or more for metallic components, compared to the outstanding acid resistance of the surface-treated filler for dental use (C-1) of Examples with a dissolution rate of less than 93 ppm.

Because there is a correlation between the results of this acid resistance test and the shear adhesion test conducted on bovine dentin, the results suggest that the surface-treated filler for dental use (C-1) of the present invention has a surface more densely coated with organic molecules, inhibiting the interaction between the filler and the polymerizable monomer having an acidic group. This likely accounts for the excellent storage stability of the dental composition even under severe conditions.

### INDUSTRIAL APPLICABILITY

A dental composition according to the present invention can be suitably used in applications such as dental cements, dental bonding materials, dental composite resins (for example, self-adhesive dental composite resins), and dental abutment construction materials in the field of dental treatment.

## Claims

1. A dental composition comprising a polymerizable monomer (A), a polymerization initiator (B), and a filler (C),
wherein the filler (C) comprises a surface-treated filler for dental use (C-1) surface-treated with a silane coupling agent (a) having a polymerizable group, and an end capping agent (b), and
the surface-treated filler for dental use (C-1) shows a dissolution rate of less than 100 ppm for metallic components in an acid resistance test.

2. The dental composition according to claim 1, wherein the end capping agent (b) is a compound having a structure represented by the following formula (1) or (2), and a boiling point of 180°C or less, 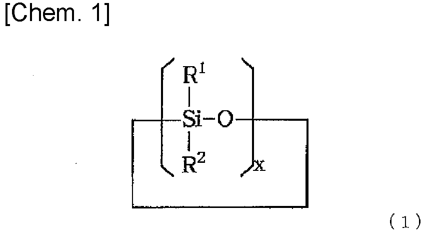 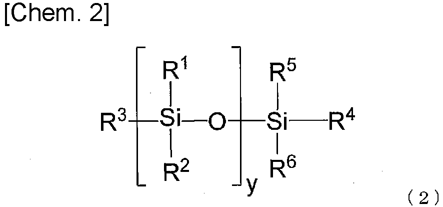 where R¹ to R⁶ each independently represent a hydrogen atom, a hydroxyl group, an alkyl group having 1 to 9 carbon atoms, or an alkoxy group having 1 to 4 carbon atoms, x represents an integer of 3 to 10, and y represents an integer of 1 to 100.

3. The dental composition according to claim 1 or 2, wherein the surface-treated filler for dental use (C-1) results from surface treatment with the end capping agent (b) following surface treatment with the silane coupling agent (a) having a polymerizable group.

4. The dental composition according to any one of claims 1 to 3, wherein the surface-treated filler for dental use (C-1) results from hydrolysis following surface treatment with the silane coupling agent (a) having a polymerizable group.

5. The dental composition according to any one of claims 1 to 4, wherein the surface-treated filler for dental use (C-1) comprises an X-ray opacity filler.

6. The dental composition according to claim 5, wherein the X-ray opacity filler results from acid treatment.

7. The dental composition according to any one of claims 1 to 6, wherein the silane coupling agent (a) having a polymerizable group comprises 3-methacryloyloxypropyltrimethoxysilane, 8-(meth)acryloyloxyoctyltrimethoxysilane, 11-(meth)acryloyloxyundecyltrimethoxysilane, and hydrolysates of these.

8. The dental composition according to any one of claims 1 to 7, wherein the end capping agent (b) is hexamethylcyclotrisiloxane.

9. The dental composition according to any one of claims 1 to 8, wherein a filler is treated with 0.01 to 20 parts by mass of the end capping agent (b) in surface treatment to obtain the surface-treated filler for dental use (C-1), relative to 100 parts by mass of the filler before surface treatment.

10. The dental composition according to any one of claims 1 to 9, wherein the polymerizable monomer (A) comprises a polymerizable monomer (A-1) having an acidic group, and a polymerizable monomer (A-2) having no acidic group.

11. The dental composition according to claim 10, wherein the content of the polymerizable monomer (A-1) having an acidic group is 1 to 40 parts by mass in 100 parts by mass of the polymerizable monomer (A).

12. A self-adhesive dental composite resin comprising a dental composition of any one of claims 1 to 11.

13. A dental bonding material comprising a dental composition of any one of claims 1 to 11.

14. A dental cement comprising a dental composition of any one of claims 1 to 11.

15. A dental composite resin comprising a dental composition of any one of claims 1 to 11.

16. A dental abutment construction material comprising a dental composition of any one of claims 1 to 11.

17. A method for producing a dental composition of any one of claims 1 to 11, comprising a step of obtaining the surface-treated filler for dental use (C-1), wherein the step of obtaining the surface-treated filler for dental use (C-1) comprises subjecting a filler to surface treatment with the silane coupling agent (a) having a polymerizable group, and subjecting the filler to surface treatment with the end capping agent (b).

18. A surface-treated filler for dental use (C-1) that results from surface treatment of filler with a silane coupling agent (a) having a polymerizable group, and an end capping agent (b),
wherein the end capping agent (b) is a compound having a structure represented by the following formula (1) or (2), and a boiling point of 180°C or less, 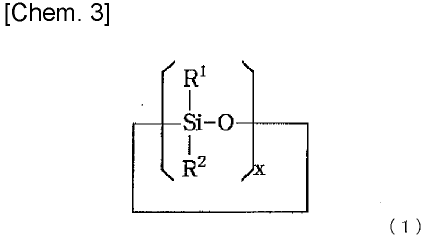 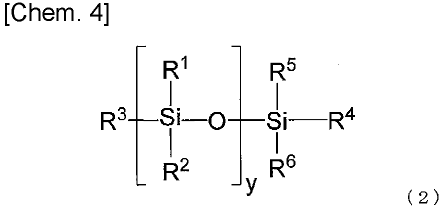 where R¹ to R⁶ each independently represent a hydrogen atom, a hydroxyl group, an alkyl group having 1 to 9 carbon atoms, or an alkoxy group having 1 to 4 carbon atoms, x represents an integer of 3 to 10, and y represents an integer of 1 to 100.

19. The surface-treated filler for dental use (C-1) according to claim 18, wherein the filler comprises an inorganic filler.

20. The surface-treated filler for dental use (C-1) according to claim 19, wherein the inorganic filler comprises an aggregate filler.

21. The surface-treated filler for dental use (C-1) according to any one of claims 18 to 20, which results from surface treatment with the end capping agent (b) following surface treatment with the silane coupling agent (a) having a polymerizable group.

22. The surface-treated filler for dental use (C-1) according to any one of claims 18 to 21, which results from hydrolysis following surface treatment with the silane coupling agent (a) having a polymerizable group.
